# EUROPEAN PATENT APPLICATION

(11) **EP 4 177 364 A1**
(43) Date of publication of application: **10.05.2023**
(21) Application number: 22187525.5
(22) Date of filing: 28.07.2022
(51) Int. Cl.: C22B 3/18, C22B 3/00, A23K 20/26, C05B 17/00, C12N 1/16, C12P 3/00

(54) **ENVIRONMENTALLY-FRIENDLY COMPOSITIONS AND METHODS FOR EXTRACTING MINERALS AND METALS FROM ORE**

(30) Priority: 08.11.2021 US 202117521290; 11.04.2022 US 202263329715 P
(71) Applicant: Locus IP Company, LLC, Solon OH 44139 (US)
(72) Inventor: Farmer, Sean, Ft. Lauderdale, FL, 33312-5536 (US); Karathur, Karthik N., Solon, OH, 44139 (US)
(74) Representative: Engelhard, Markus

(57) **Abstract**

The subject invention provides safe, environmentally-friendly, compositions and methods for extracting minerals and/or metals from ore. More specifically, the subject invention provides for bioleaching using a composition comprising one or more biosurfactant-producing microorganisms and/or microbial growth by-products. In specific embodiments, the composition comprises biosurfactant-producing yeasts and/or their growth by-products.

## Description

### BACKGROUND OF THE INVENTION

"Leaching" is a widely used extractive metallurgy technique that converts metals found in ore (pieces of mineral, rock or soil) into soluble salts in aqueous media. There are a variety of leaching processes, usually classified by the types of reagents used. These techniques utilize either chemicals or microorganisms to extract metals, depending upon the ores or materials to be processed.

One leaching technique, called heap leach mining provides a low-cost method of extracting metal values from relatively low-grade metal-bearing materials, and has found particular application in the processing of metal-bearing ores. Heap leach mining uses a series of chemical reactions that absorb specific minerals and then re-separates them after their division from other earth materials in the ore. Generally, an ore is mined, crushed, and then transported to a heap location where it is stacked onto an impervious liner, or heap leach pad.

The ore is continuously sprayed or irrigated with a suitable solution, or process fluid. Process fluids can be alkaline or acidic. For example, a dilute alkaline cyanide solution, ozone and sulfuric acid can be used, depending on the substrate being extracted.

The process fluid extracts metals in the ore upon contact with the ore and the resulting "pregnant solution" trickles slowly under the force of gravity to the pad. The heap leach pad typically has a sloped base to allow pregnant solution to flow into collection drains for separating the metal from the process fluid. The percolation of solution through the heap is called the "leach cycle," which can take from one or two months for simple oxide ores (e.g., most gold ores) up to two years (for nickel laterite ores).

After separating the precious metals from the pregnant solution, the dilute cyanide solution (now called "barren solution") is normally re-used in the heap-leach-process or sent to an industrial water treatment facility, where the residual cyanide or acid is treated and residual metals are removed.

Although heap leaching can be a low-cost process, normal recovery rates range from 60-70%, although there are exceptions. It is normally most profitable with low-grade ores. Higher-grade ores are usually put through more complex milling processes, where higher recoveries justify the extra cost. The process chosen depends on the properties of the ore.

Additionally, for the leaching of certain precious metals, such as gold and silver, leaching reagents include cyanide, thiosulfate, thiocyanate, halides and halogens (e.g., chlorides, bromides, iodides, chlorine, bromine and iodide), and thiourea. Of these, cyanide remains the predominant reagent applied on industrial scales for gold and gold-silver ores. Halides (chlorine and chlorides in particular) are often used in the final refining of impure bullion (bars, coins or ingots of a metal). Furthermore, metallic mercury, which is highly poisonous and environmentally hazardous, is still used by many artisanal miners.

Despite being a robust leaching reagent (or lixiviant), use of sodium cyanide, and cyanides of other alkali metals (e.g., potassium) and alkali earth metals (e.g., calcium), poses a number of challenges, principally due to its toxicity, regulatory restrictions, high carbon footprint and low selectivity in low grade ores. It is particularly problematic for gold ores with high copper and/or high silver content, as copper is often present at levels of around 1000 times the gold concentration, leading to excessive cyanide consumption, and removal of available cyanide for gold leaching. Cyanide is also an expensive reagent, so that using it for lower value metals such as copper (and less so, for silver) quickly becomes uneconomic, not only for leaching, but also due to downstream impacts. In addition, it generates weak acid dissociable (WAD) cyanides, which further require cyanide detoxification/destruction or recovery processes.

Heap leaching can be used for extracting, for example, nickel, copper and gold. Leaching of certain other compounds often involves the use of pressurized vessels, called autoclaves. Cobalt, for example, is commonly produced using high pressure acid leaching (HPAL). The process utilizes temperatures around 255°C and pressures around 725 psi, in addition to sulfuric acid to separate the metal from the ore.

In HPAL, the ore is mined and crushed to create a fine material, which is mixed with water to create a slurry. The slurry is heated and pumped into an autoclave, to which acid is added. The slurry and acid then react as they flow through several compartments within the autoclave. It takes approximately 60 minutes to complete the leaching process in the autoclave. Upon leaving the high pressure and temperature atmosphere of the autoclave, the slurry must be returned to atmospheric conditions. This is accomplished through two or more letdown/flash stages. Once the slurry is at atmospheric conditions it is washed and separated, at which point the metal can be recovered from the liquid fraction.

Leaching processes may be enhanced by the use of microorganisms, such as thermophilic and/or acidophilic bacteria that grow on the surface and in the cracks of ore fragments. This process of "bio-stimulation" can provide, for example, catalyzation of oxidation reactions within the ore. Such a process typically involves use of a high concentration of microbes.

Microorganisms themselves can also be used to carry out leaching, through "bioleaching." Typically, bioleaching requires a slurry containing carbon dioxide and other microbial nutrients, sulfide concentrate and microbes, as well as tanks, microbial monitoring systems, and control of pH and redox potential.

Biological leaching processes are more environmentally-friendly alternatives to conventional smelting processes, which discharge large amounts of carbon dioxide, sulfur dioxide, and various toxic materials, such as arsenic, into the environment. Currently known bio-stimulation and bioleaching processes have a number of disadvantages, however. They can be time consuming and cost-inefficient. For example, bioleaching of copper concentrates is typically very slow, with incomplete recoveries achieved even after many weeks to months of leaching.

Additionally, it can be costly and cumbersome to transport and keep microbes viable at a site of application. Currently, microbe-based leaching operations are limited to sourcing microbial amendments from far-flung producers whose product quality suffers due to upstream processing delays, methods used to stabilize the product for future distribution, supply chain bottlenecks, improper storage, and other factors that inhibit the timely delivery and application of viable, high cell-count microbial products.

Accordingly, there is a need for improved compositions and methods for extracting valuable minerals and/or metals from ore.

Phosphate ore is an essential raw material for manufacturing phosphoric industrial products. It has been widely utilized in agriculture, as well as in the pharmaceutical, chemical, and food industries. There are four major types of phosphate resources based on mineralization: igneous deposits, metamorphic deposits, sedimentary deposits and biogenic deposits (e.g., guano accumulations). A majority of phosphate resources is of sedimentary origin. There is no synthetic substitute for phosphate, which makes the responsible and sustainable mining of phosphate deposits worldwide vital to the health and wellbeing of our global population.

Phosphate rock, when used in an untreated form, is not very soluble and provides little available phosphorus to plants, except in some moist acidic soils. Treating phosphate rock with, for example, sulfuric acid produces phosphoric acid, the water-soluble material from which most phosphate fertilizers are derived. The vast majority of phosphoric acid is used in the production of agricultural fertilizers, but phosphoric acid is also used as an additive in livestock feed and even in food products.

When phosphate is brought out of the ground it is naturally associated with unwanted material called gangue, mainly quartz, mica, feldspar, dolomite, calcite, and clays. Gangue is removed from the phosphate ore through beneficiation, a process typically involving screening, washing and/or flotation, to physically separate out different categories of material.

Phosphate rocks also contain a variety of heavy metals, such as Cr, Cd, Cu, Mn, Ni, U, and Zn. Cadmium, in particular, is a toxic heavy metal component of phosphate rock deposits.

In the production of fertilizers, phosphate rock is processed using acidulation, wherein the phosphate is treated with a mineral acid such as sulfuric acid, hydrochloric acid or nitric acid, thereby producing a phosphoric acid. This phosphoric acid can contain varying levels of cadmium in a dissolved state, which can ultimately end up accumulating in soil, plant matter, and in food products. This can have detrimental long-term effects on human, plant and animal health.

Currently, two main approaches exist for mitigating this problem. First is the removal of cadmium and other heavy metals from soil after application of the fertilizer. This often involves soil washing using extracting agents, such as acids, bases, chelators, electrolytes, oxidizing agents and surfactants. However, many of these extracting agents can alter the productivity of soil by changing the chemical, physical and mineral properties of the soil.

The second approach is to remove the cadmium prior to its conversion into a fertilizer product. Decadmiation of phosphate rock prior to acidulation can be carried out using a process called calcination. Calcination involves heating the phosphate rock to extremely high temperatures (e.g., over 700°C), which essentially boils the cadmium to the point of volatilization. Aside from the high energy expenditure required to obtain the proper temperature, this process can change the structure and reduce the reactivity of the phosphate rock. This can in turn reduce the value of the rock for producing fertilizers. Some methods utilize microwaves to achieve a similar evaporation of cadmium; however, these methods are mostly limited to the laboratory scale.

A less energy intensive, yet potentially less effective method for decadmiation of phosphate rock involves washing pulverized phosphate ore with an extracting agent, such as sodium EDTA, ammonium acetate, and hydrochloric acid; however, this method can require multiple extractions to achieve satisfactory cadmium removal.

Various methods of purifying wet-process phosphoric acid with respect to its heavy metal content are also known to the art. These include, for example, co-crystallization with anhydrite, precipitation with sulfide ions, removal by solvent extraction, removal by ion exchange, and laboratory scale separation by membrane technology.

Co-crystallization or co-precipitation of cadmium with gypsum is useful because cadmium shows great affinity to anhydrite (Ca₂SO₄). The result, however, is a significant production of phosphogypsum waste, a radioactive water pollutant.

Some heavy metals can be readily precipitated out and removed from the phosphoric acid, by, for example, adding hydrogen sulfide, sodium sulfide solution, potassium sulfide solution or an ammonium sulfide solution, or by adding calcium and barium sulfide. The cadmium precipitates out as cadmium sulfide; however, cadmium is much more difficult to precipitate out than other heavy metals, often requiring a high heat, high pressure process to, for example, reduce the solubility of the cadmium compounds formed in the phosphoric acid. Additionally, the hydrogen sulfide by-products of these processes are respiratory toxins.

With ion exchange, both cation and anion exchangers can be used. For example, in reductive conditions, cadmium can complex with bromium, chloride or iodine, which exhibit high affinity to anionic resins. These methods, however, require pre-removal of insoluble substances to ensure proper efficiency.

Finally, solvent extraction using, for example, an amino halide, is more useful for other metal impurities present in greater quantities, such as iron, calcium and magnesium. With low selectivity for cadmium, the process must be repeated several times to achieve a significant reduction of cadmium. Furthermore, the solvent must be recovered and treated afterwards.

As described above, safe and efficient recovery of phosphorus from ore is important for human, plant and animal health. Because of growing concerns over heavy metal impurities, such as cadmium, accumulating in soils, and because of the often inefficient, chemical- and energy-intensive methods presently used in the art, improved methods are needed for the processing of phosphorous ore and phosphoric acid.

### BRIEF SUMMARY OF THE INVENTION

The subject invention relates generally to metals recovery. More specifically, the subject invention relates to microbes, as well as by-products of their growth, such as biosurfactants, solvents, and/or enzymes, for use in bioleaching.

In specific embodiments, the subject invention provides microbe-based compositions and methods for recovering valuable minerals and/or metals, such as, e.g., gold, copper, silver, lithium and cobalt, from ore and/or mine tailings. These compositions, and the methods of their use, are safe, environmentally-friendly and cost-efficient.

In preferred embodiments, the microbe-based composition of the subject invention is an environmentally-friendly biological leaching reagent comprising one or more microorganisms and/or microbial growth by-products. In one embodiment, at least one of the microorganisms is a biosurfactant-producing yeast.

In one embodiment, the microbial growth by-products are biosurfactants, enzymes, proteins, peptides, amino acids and/or solvents. In one embodiment, the microbial growth by-products are biosurfactants. Biosurfactants according to the subject invention include, for example, low-molecular-weight glycolipids, cellobiose lipids, lipopeptides, flavolipids, phospholipids, and high-molecular-weight polymers such as lipoproteins, lipopolysaccharide-protein complexes, and/or polysaccharide-protein-fatty acid complexes.

In some embodiments, the growth by-product is produced by the one or more microorganisms present in the composition. In some embodiments, the growth by-product is added to the composition, either in crude or purified form, in addition to any growth by-products that are produced by the microorganisms.

In certain specific embodiments, the biological leaching reagent comprises a yeast fermentation product comprising yeast cell biomass and growth by-products thereof in fermentation medium in which the yeast was produced. Preferably, the yeast is a biosurfactant-producing yeast. Even more preferably, the yeast is *Starmerella bombicola,* which is capable of producing glycolipid biosurfactants, e.g., sophorolipids (SLP), at high concentrations.

In some embodiments, the yeast fermentation product is obtained during production of biosurfactants. During submerged cultivation of a biosurfactant-producing microorganism, biosurfactants are excreted into the fermentation broth. The biological leaching reagent can comprise the entire broth containing microbes, biosurfactants and other growth by-products, such as, e.g., excreted metabolites and/or cell wall components.

Alternatively, the biosurfactants can be harvested from the broth for further processing and/or purification. What remains after harvesting the biosurfactants is a supernatant comprising yeast cell biomass, residual biosurfactants and other growth by-products, such as, e.g., excreted metabolites and/or cell wall components. In certain embodiments, the biological leaching reagent of the subject invention comprises this supernatant.

In certain embodiments, use of yeast fermentation products in the biological leaching reagents can be superior to, for example, purified microbial metabolites alone, due to, for example, the advantageous properties of yeast cells. These properties include, for example, high concentrations of mannoprotein and/or beta-glucan in and/or on the yeast cell wall. These compounds can serve as, for example, effective emulsifiers. Additionally, the yeast fermentation product can further comprise biosurfactants, other metabolites, and/or cellular or extracellular components that are present in the culture, such as, e.g., solvents, acids, vitamins, minerals, enzymes, proteins, peptides, amino acids and others (e.g., lactic acid, ethanol, etc.), in the culture.

In one embodiment, the biological leaching reagent can be enhanced with additional components as are needed, depending upon, for example, the ore type, mineral type, volume of ore, and other factors. These enhancing components can include additional microbial cultures, such as yeast and/or bacterial cultures. The enhancing components can also include additional pure or crude form biosurfactants, acids, solvents, enzymes, proteins, peptides, amino acids and/or other metabolites.

In some embodiments, the additional microbial cultures comprise biosurfactant-producers, such as, for example, *Wickerhamomyces anomalus, Pseudozyma aphidis, Saccharomyces cerevisiae, Pichia guilliermondii, Bacillus subtilis, Bacillus amyloliquefaciens, Bacillus licheniformis, Pseudomonas aeruginosa, Streptococcus* spp., and many others.

In some embodiments, the additional microbial cultures comprise microorganisms capable of accumulating nanoparticles of minerals and/or metals such as, for example, copper, cobalt, lithium, gold and/or silver, by solubilizing the metal present in ore to a soluble ionic form and converting it into nanoparticles within their cells. For example, thermophilic and/or acidophilic bacteria such as *Cupriavidus metallidurans,* which can precipitate nanoparticles of gold, can be added to the biological leaching reagent as an enhancement.

In some embodiments, the enhancing components comprise additional biosurfactants. The biosurfactants can be added as part of a microbial culture, or as a crude or purified form after being extracted from a microbial culture.

The biosurfactants can comprise glycolipids such as, for example, rhamnolipids (RLP), sophorolipids (SLP), trehalose lipids or mannosylerythritol lipids (MEL). In one embodiment, the biosurfactants are lipopeptides, such as, e.g., surfactin, iturin, fengycin, viscosin and/or lichenysin. In one embodiment, the biosurfactants are polymeric biosurfactants, such as, for example, emulsan, lipomanan, alasan, and/or liposan. In one embodiment, the biological leaching reagent comprises, and/or is enhanced by the addition of, more than one biosurfactant and/or biosurfactant derivative.

In certain embodiments, the subject invention provides a method for extracting valuable minerals and/or metals from ore, wherein the method comprises obtaining ore from, e.g., an ore deposit, said ore comprising one or more valuable minerals and/or metals, in addition to gangue; applying a biological leaching reagent comprising one or more microorganisms and/or microbial growth by-products, to the ore; allowing the valuable minerals and/or metals to separate from the ore; and collecting the valuable minerals and/or metals. In some embodiments, the method is performed in a tank, vat, column (e.g., unsaturated or saturated leaching column) or pool.

In one embodiment, the ore is mined and crushed, micronized, pulverized or ground into smaller particles. In one embodiment, the ore is in the form of mine tailings, or the waste products left behind after a mineral has been separated from gangue.

In a specific embodiment, the method comprises applying the biological leaching reagent in liquid form to the smaller ore particles, and mixing the particles and biological leaching reagent to form a liquid slurry.

The slurry can then be left for any amount of time sufficient to leach the valuable mineral and/or metal particles from the ore. The slurry can optionally be mixed and/or circulated continuously (e.g., mechanically or using aeration) throughout the leaching time period to ensure that maximum contact is made between the ore particles and the leaching reagent.

In one embodiment, the mineral particles are sequestered by the cells of the microorganism(s) of the biological leaching reagent. In one embodiment, the mineral particles separate from the ore and are dispersed and/or float in the liquid as solution. The liquid fraction of the slurry can be siphoned, drained, filtered, or otherwise removed. The mineral particles present in the liquid can be washed to remove residual microbial cell matter, collected, and dried, incinerated, and/or processed by any other means known in the metallurgical arts.

In some embodiments, the method comprises applying the biological leaching reagent in liquid form to a pile or column filled with the crushed ore particles, and allowing the biological leaching reagent to percolate through the particles to a collection apparatus using gravity. In some embodiments, the method can be used for bio-stimulation of heap leaching processes.

The biological leaching reagent can enhance recovery of valuable minerals and/or metals from ore due to, for example, microbial sequestration activity and/or metabolites that solubilize the minerals and/or metals from the ore.

The microbes can be live (or viable), or inactive at the time of application. In certain embodiments, the microorganisms can grow *in situ* and produce active compounds (e.g., metabolites) onsite. Consequently, a high concentration of desirable metabolites (e.g., biosurfactants, solvents, enzymes, proteins, peptides and amino acids) and the microorganisms that produce them can be achieved easily and continuously at a treatment site (e.g., an ore mining site or a heap leaching pile).

The method can further comprise adding materials to enhance microbe growth during application (e.g., adding nutrients).

The method can further comprise adding additional materials to enhance extraction of the valuable minerals and/or metals, for example, additional microbial cultures, such as yeast and/or bacterial cultures and/or additional pure or crude form biosurfactants, acids, solvents, enzymes, proteins, peptides and/or amino acids.

Advantageously, in certain embodiments, the methods take as little as a few hours, e.g., 3 to 12 hours, to one day to leach the minerals from the ore. The amount of time, however, depends upon, for example, how finely ground the ore particles are, the volume of ore particles being processed, and what types and/or combinations of microorganisms and other components are used in the biological leaching reagent.

Additionally, in one embodiment, the subject methods reduce the amount of refining and processing needed to recover pure or nearly pure metals from ore. For example, the subject invention can be used to separate the metals in a doré bar and reduce the amount of refining needed to do so.

The method can be carried out at atmospheric pressure and lower temperatures than traditional metal smelting operations. Thus, the method does not require complicated equipment or high energy consumption, and cultivation of the biological leaching reagent used in the subject method can be performed on site, for example, at a mine or at a leaching site.

Advantageously, the present invention can be used without releasing large quantities of inorganic and toxic compounds into the environment. Additionally, the compositions and methods utilize components that are biodegradable and toxicologically safe, and can be used to reduce the amount of toxic waste produced during mining and leaching processes.

The subject invention further relates generally to the removal of metal impurities from ore, wherein the ore itself is the more valuable or desirable portion. More specifically, the subject invention provides environmentally-friendly compositions and methods for extracting metal impurities, such as, for example, cadmium, from, for example, phosphate ore (rock) and/or phosphoric acid. In certain embodiments, the metal impurities can be safely discarded using methods known in the art, while in other embodiments, the metal impurities can be recycled and/or processed for other uses to reduce waste and pollution.

Advantageously, the compositions and methods of the subject invention reduce the energy input and chemical usage required for processing phosphate ore into useful products, such as fertilizers and animal feed supplements. Accordingly, the subject invention is useful for improving the safety, and reducing the air, ground and water pollution, of processing mined phosphate ore.

In certain embodiments, the subject invention provides decadmiation compositions comprising components that are derived from microorganisms. In certain embodiments, the decadmiation composition comprises a microbial biosurfactant. In certain embodiments, the composition comprises a biosurfactant, an acid, and, optionally, a pH adjuster.

Use of the term "decadmiation" composition is not intended to limit the composition's usefulness to extracting or removing only cadmium from a substance. Rather, the subject invention further facilitates the extraction of other metal impurities, such as, for example, lead, nickel, arsenic, copper, and vanadium.

In certain embodiments, the biosurfactant of the decadmiation composition is utilized in crude form. The crude form can comprise, in addition to the biosurfactant, fermentation broth in which a biosurfactant-producing microorganism was cultivated, residual microbial cell matter or live or inactive microbial cells, residual nutrients, and/or other microbial growth by-products.

In some embodiments, the biosurfactant is utilized after being extracted from a fermentation broth and, optionally, purified.

The biosurfactant according to the subject invention can be a glycolipid (e.g., sophorolipids, rhamnolipids, cellobiose lipids, mannosylerythritol lipids and trehalose lipids), lipopeptide (e.g., surfactin, iturin, fengycin, arthrofactin and lichenysin), flavolipid, phospholipid (e.g., cardiolipins), fatty acid ester compound, fatty acid ether compound, and/or high molecular weight polymers such as lipoproteins, lipopolysaccharide-protein complexes, and polysaccharide-protein-fatty acid complexes.

In certain specific embodiments, the biosurfactant is a sophorolipid (SLP), including linear SLP, lactonic SLP, acetylated SLP, de-acetylated SLP, salt-form SLP, esterified SLP derivatives, amino acid-SLP conjugates, and other SLP derivatives or isomers that exist in nature and/or are produced synthetically. In preferred embodiments, the SLP is a linear SLP or a derivatized linear SLP.

In certain embodiments, the acid of the decadmiation composition is an organic acid, such as, for example, acetic acid, citric acid, lactic acid, butyric acid, sorbic acid, benzoic acid, formic acid, fumaric acid, propionic acid, ascorbic acid, glyoxylic acid, malonic acid, pyruvic acid, oxalic acid, uric acid, malic acid, tartaric acid and/or analogs thereof. In certain embodiments, the acid is selected from inorganic acids such as, for example, sulfuric acid, nitric acid, phosphoric acid, hydrochloric acid, boric acid and analogs thereof. In preferred embodiments, the acid is citric acid.

In certain embodiments, the acid is one that is characterized as a "weak" acid. Advantageously, in certain embodiments, the use of a weaker acid is effective for removal of impurities from an ore while retaining the integrity and value of the ore being treated.

In certain embodiments, the phosphate in the ore serves to buffer the acid and stabilize the formulation at pH 2 to 6, preferably about pH 4; however, optional additional pH adjusters can be included in the composition to adjust and/or stabilize the pH within the desired range, for example, sodium hydroxide or potassium hydroxide.

In certain embodiments, the subject invention provides a method for extracting metals and other impurities from phosphate ore, wherein the method comprises (i) obtaining the phosphate ore, said ore comprising phosphate and an impurity; (ii) contacting a decadmiation composition according to the subject invention with the ore for a period of time to yield a mixture comprising a treated phosphate ore and an impurity that has reacted with the decadmiation composition; and (iii) separating the reacted impurity and decadmiation composition from the mixture.

The method can be carried out in a heap leach pad, a column, or any other laboratory or industrial sized reactor.

In some embodiments, (iii) comprises applying a washing fluid comprising water and, optionally, an organic solvent, to the mixture, wherein the washing fluid comprises the reacted impurity and decadmiation composition and wherein the phosphate ore remains a solid that is decanted and filtered out of the fluid. In some embodiments, the solvent is an alcohol, such as ethanol. Step (iii) can be repeated as many times as necessary to achieve a desired reduction in impurity content.

In certain embodiments, the method comprises (a) obtaining the phosphate ore, said ore comprising phosphate and an impurity; (b) preparing a slurry of the phosphate ore in water and maintaining the slurry under agitation; (c) applying a decadmiation composition according to the subject invention to the slurry under agitation for a period of time to yield a mixture comprising a treated phosphate ore and an impurity that has reacted with the decadmiation composition; (d) halting the agitation and allowing the mixture to stand for another period of time, thereby causing the formation of a precipitate comprising the treated phosphate ore and an aqueous layer comprising the reacted impurity and decadmiation composition; and (e) separating the precipitate from the aqueous layer.

In some embodiments, (e) further comprises applying a washing fluid comprising water and, optionally, an organic solvent, to the separated precipitate to further remove reacted impurity and decadmiation composition from the precipitate. Step (e) can be repeated as many times as necessary to achieve a desired reduction in impurity content.

In certain embodiments, the decadmiation composition according to the subject invention is effective due to amphiphiles-mediated penetration of the phosphate ore with an acid. In some embodiments, the sophorolipid or other biosurfactant serves as a vehicle for facilitating the transport of acid molecules into nanoscale pores within the ore. For example, in some embodiments, a sophorolipid will form a micelle containing the acid, wherein the micelle is less than 100 nm, less than 50 nm, less than 25 nm, less than 15 nm or less than 10 nm in size. The small size and amphiphilic properties of the micelle allow for enhanced penetration into the ore so that greater contact can be made with impurities therein.

In certain embodiments, the impurity is as cadmium, nickel, arsenic, iron, vanadium, mercury, copper or lead. In certain preferred embodiments, the impurity is cadmium; however, in some embodiments, more than one impurity is present, wherein the total content of each of the more than one impurity is reduced according to the subject methods.

In certain embodiments the methods of the subject invention result in at least 25% reduction in impurities content, preferably at least 50% reduction after one treatment. In come embodiments, the phosphate ore can be treated multiple times to further reduce the impurities content.

In one embodiment, the method comprises crushing, grinding or pulverizing the phosphate ore into smaller particles, for example, less than 500 nm in size, prior to treating with the decadmiation composition.

In some embodiments, the phosphate ore is obtained from an ore deposit in a raw form. This raw form can comprise additional materials, or gangue. Thus, in certain embodiments, the method can further comprise, after obtaining the phosphate ore, subjecting the ore to one or more beneficiation processes to liberate the phosphate ore from gangue material. The one or more beneficiation processes can include, for example, comminution, scrubbing, washing, screening, flotation, and/or hydrocycloning.

In some embodiments, the method comprises oxidizing the organic matter present in the phosphate ore using, for example, hydrogen peroxide, prior to contacting the phosphate ore with the decadmiation composition.

In some embodiments, the method comprises applying the decadmiation composition to wet process phosphoric acid produced after phosphate ore has undergone acidulation (e.g., reaction with sulfuric acid). In certain embodiments, the decadmiation composition can react with dissolved cadmium and other impurities present in the wet process phosphoric acid, causing the impurities to precipitate out of the liquid for collection and removal.

Advantageously, in certain embodiments, the decadmiation composition according to the subject invention has comparable effectiveness to traditional chemical extracting agents, but with reduced negative environmental impacts. Furthermore, the methods of the subject invention do not require complicated equipment or high energy consumption, and production of the decadmiation composition can be performed on site, for example, at an ore mine or at a leaching site.

### DETAILED DESCRIPTION

The subject invention relates generally to metals recovery and/or removal. More specifically, the subject invention relates to microbes, as well as by-products of their growth, such as biosurfactants, solvents, and/or enzymes, for use in bioleaching.

In specific embodiments, the subject invention provides microbe-based compositions and methods for recovering valuable minerals and/or metals, such as, e.g., gold, copper, silver, lithium and cobalt, from ore and/or mine tailings. These compositions, and the methods of their use are safe, environmentally-friendly and cost-efficient.

In preferred embodiments, the microbe-based composition of the subject invention is an environmentally-friendly biological leaching reagent comprising one or more microorganisms and/or microbial growth by-products. In one embodiment, at least one of the microorganisms is a biosurfactant-producing yeast.

In certain embodiments, the subject invention provides a method for extracting valuable minerals and/or metals from ore, wherein the method comprises obtaining ore from, e.g., an ore deposit, said ore comprising one or more valuable minerals and/or metals, in addition to gangue; applying a biological leaching reagent comprising one or more microorganisms and/or microbial growth by-products, to the ore; allowing the valuable minerals and/or metals to separate from the ore; and collecting the valuable minerals and/or metals.

The biological leaching reagent can enhance recovery of valuable minerals and/or metals from ore due to, for example, microbial sequestration activity and/or metabolites that sequester nanoparticles of the minerals and/or metals.

The subject invention further relates generally to the removal of metal impurities from phosphate ore. More specifically, the subject invention provides environmentally-friendly compositions and methods for extracting metal impurities, such as, for example, cadmium, from phosphate ore (rock) and/or phosphoric acid. In certain embodiments, the metal impurities can be safely discarded using methods known in the art, while in other embodiments the metal impurities can be recycled and/or processed for other uses to reduce waste and pollution.

Advantageously, the compositions and methods of the subject invention reduce the energy input and chemical usage required for processing phosphate ore into useful products, such as fertilizers and animal feed supplements. Accordingly, the subject invention is useful for improving the safety, and reducing the air, ground and water pollution, of processing mined phosphate ore.

### Selected Definitions

As used herein, reference to a "microbe-based composition" means a composition that comprises components that were produced as the result of the growth of microorganisms or other cell cultures. Thus, the microbe-based composition may comprise the microbes themselves and/or by-products of microbial growth. The microbes may be in a vegetative state, in spore form, in mycelial form, in any other form of propagule, or a mixture of these. The microbes may be planktonic or in a biofilm form, or a mixture of both. The by-products of growth may be, for example, metabolites, cell membrane components, expressed proteins, and/or other cellular components. The microbes may be intact or lysed. In some embodiments, the microbes are present, with broth in which they were grown, in the microbe-based composition. The cells may be present at, for example, a concentration of 1 × 10⁴, 1 × 10⁵, 1 × 10⁶, 1 × 10⁷, 1 × 10⁸,1 × 10⁹, 1 × 10¹⁰, 1 × 10¹¹, 1 × 10¹², 1 × 10¹³ or more propagules per milliliter of the composition. As used herein, a propagule is any portion of a microorganism from which a new and/or mature organism can develop, including but not limited to, cells, spores, conidia, hyphae, mycelia, cysts, buds and seeds.

The subject invention further provides "microbe-based products," which are products that are to be applied in practice to achieve a desired result. The microbe-based product can be simply the microbe-based composition harvested from the microbe cultivation process. Alternatively, the microbe-based product may comprise further ingredients that have been added. These additional ingredients can include, for example, stabilizers, buffers, appropriate carriers, such as water, salt solutions, or any other appropriate carrier, added nutrients to support further microbial growth, non-nutrient growth enhancers, such as plant hormones, and/or agents that facilitate tracking of the microbes and/or the composition in the environment to which it is applied. The microbe-based product may also comprise mixtures of microbe-based compositions. The microbe-based product may also comprise one or more components of a microbe-based composition that have been processed in some way such as, but not limited to, filtering, centrifugation, lysing, drying, purification and the like.

As used herein, "harvested" refers to removing some or all of a microbe-based composition from a growth vessel.

As used herein, a "biofilm" is a complex aggregate of microorganisms, such as bacteria, wherein the cells adhere to each other on a surface. The cells in biofilms are physiologically distinct from planktonic cells of the same organism, which are single cells that can float or swim in liquid medium.

As used herein, an "isolated" or "purified" nucleic acid molecule, polynucleotide, polypeptide, protein or organic compound such as a small molecule (e.g., those described below), is substantially free of other compounds, such as cellular material, with which it is associated in nature. A purified or isolated polynucleotide (ribonucleic acid (RNA) or deoxyribonucleic acid (DNA)) is free of the genes or sequences that flank it in its naturally-occurring state. A purified or isolated polypeptide is free of the amino acids or sequences that flank it in its naturally-occurring state. A purified or isolated microbial strain means that the strain is removed from the environment in which it exists in nature. Thus, the isolated strain may exist as, for example, a biologically pure culture, or as spores (or other forms of the strain) in association with a carrier.

In certain embodiments, purified compounds are at least 60% by weight (dry weight) the compound of interest. Preferably, the preparation is at least 75%, more preferably at least 90%, and most preferably at least 99%, by weight the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis.

A "metabolite" refers to any substance produced by metabolism (e.g., a growth by-product) or a substance necessary for taking part in a particular metabolic process. A metabolite can be an organic compound that is a starting material (e.g., glucose), an intermediate (e.g., acetyl-CoA) in, or an end product (e.g., n-butanol) of metabolism. Examples of metabolites include, but are not limited to, biopolymers, enzymes, toxins, acids, solvents, alcohols, proteins, peptides, amino acids, vitamins, minerals, microelements, and biosurfactants.

By "reduces" is meant a negative alteration, and by "increases" is meant a positive alteration, wherein the alteration is at least 0.001%, 0.01%, 0.1%, 1%, 5%, 10%, 15%, 20%, 25%, 30%, 35%, 45%, 50%, 55%, 60%, 65%, 70%, 75%, 80%, 85%, 90%, 95%, 99%, or 100%, inclusive of all values therebetween.

By "reference" is meant a standard or control condition.

By "salt-tolerant" is meant a microbial strain capable of growing in a sodium chloride concentration of fifteen (15) percent or greater. In a specific embodiment, "salt-tolerant" refers to the ability to grow in 150 g/L or more of NaCl.

As used herein, "surfactant" means a compound that lowers the surface tension (or interfacial tension) between two liquids or between a liquid and a solid. Surfactants act as, e.g., detergents, wetting agents, emulsifiers, foaming agents, and/or dispersants. A "biosurfactant" is a surface-active substance produced by a living cell and/or using naturally-derived substrates.

Biosurfactants are a structurally diverse group of surface-active substances consisting of two parts: a polar (hydrophilic) moiety and non-polar (hydrophobic) group. Due to their amphiphilic structure, biosurfactants can, for example, increase the surface area of hydrophobic water-insoluble substances, increase the water bioavailability of such substances, and change the properties of bacterial cell surfaces. Biosurfactants can also reduce the interfacial tension between water and oil and, therefore, lower the hydrostatic pressure required to move entrapped liquid to overcome the capillary effect. Biosurfactants accumulate at interfaces, thus reducing interfacial tension and leading to the formation of aggregated micellar structures in solution. The formation of micelles provides a physical mechanism to mobilize, for example, oil in a moving aqueous phase.

The ability of biosurfactants to form pores and destabilize biological membranes also permits their use as antibacterial, antifungal, and hemolytic agents to, for example, control pests and/or microbial growth.

Typically, the hydrophilic group of a biosurfactant is a sugar (e.g., a mono-, di-, or polysaccharide) or a peptide, while the hydrophobic group is typically a fatty acid. Thus, there are countless potential variations of biosurfactant molecules based on, for example, type of sugar, number of sugars, size of peptides, which amino acids are present in the peptides, fatty acid length, saturation of fatty acids, additional acetylation, additional functional groups, esterification, polarity and charge of the molecule.

These variations lead to a group of molecules comprising a wide variety of classes, including, for example, glycolipids (e.g., sophorolipids, rhamnolipids, cellobiose lipids, mannosylerythritol lipids and trehalose lipids), lipopeptides (e.g., surfactin, iturin, fengycin, arthrofactin and lichenysin), flavolipids, phospholipids (e.g., cardiolipins), fatty acid ester compounds, and high molecular weight polymers such as lipoproteins, lipopolysaccharide-protein complexes, and polysaccharide-protein-fatty acid complexes. Each type of biosurfactant within each class can further comprise subtypes having further modified structures.

Like chemical surfactants, each biosurfactant molecule has its own HLB value depending on its structure; however, unlike production of chemical surfactants, which results in a single molecule with a single HLB value or range, one cycle of biosurfactant production typically results in a mixture of biosurfactant molecules (e.g., subtypes and isomers thereof).

The phrases "biosurfactant" and "biosurfactant molecule" include all forms, analogs, orthologs, isomers, and natural and/or anthropogenic modifications of any biosurfactant class (e.g., glycolipid) and/or subtype thereof (e.g., sophorolipid).

As used herein, the term "sophorolipid," "sophorolipid molecule," "SLP" or "SLP molecule" includes all forms, and isomers thereof, of SLP molecules, including, for example, acidic (linear) SLP (ASL) and lactonic SLP (LSL). Further included are mono-acetylated SLP, di-acetylated SLP, esterified SLP, SLP with varying hydrophobic chain lengths, cationic and/or anionic SLP with fatty acid-amino acid complexes attached, esterified SLP, SLP-metal complexes, SLP-salt derivatives (e.g., a sodium salt of a linear SLP), and other, including those that are and/or are not described within in this disclosure.

In preferred embodiments, the SLP molecules according to the subject invention are represented by General Formula (1) and/or General Formula (2), and are obtained as a collection of 30 or more types of structural homologues having different fatty acid chain lengths (R³), and, in some instances, having an acetylation or protonation at R¹ and/or R².

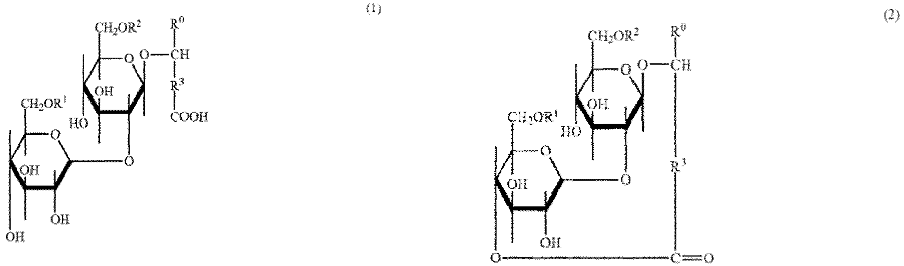

In General Formula (1) or (2), R⁰ can be either a hydrogen atom or a methyl group. R¹ and R² are each independently a hydrogen atom or an acetyl group. R³ is a saturated aliphatic hydrocarbon chain, or an unsaturated aliphatic hydrocarbon chain having at least one double bond, and may have one or more Substituents.

Examples of the Substituents include halogen atoms, hydroxyl, lower (C1-6) alkyl groups, halo lower (C1-6) alkyl groups, hydroxy lower (C1-6) alkyl groups, halo lower (C1-6) alkoxy groups, and others. R³ typically has 11 to 20 carbon atoms. In certain embodiments of the subject invention, R³ has 18 carbon atoms.

SLP are typically produced by yeasts, such as *Starmerella* spp. yeasts and/or *Candida* spp. yeasts, e.g., *Starmerella (Candida) bombicola, Candida apicola, Candida batistae, Candida floricola, Candida riodocensis, Candida stellate* and/or *Candida kuoi.* SLP have environmental compatibility, high biodegradability, low toxicity, high selectivity and specific activity in a broad range of temperature, pH and salinity conditions. Additionally, in some embodiments, SLP can be advantageous due to their small micelle size, which can help facilitate the movement of the micelle, and compounds enclosed therein, through nanoscale pores and spaces. In certain embodiments, the micelle size of a SLP is less than 100 nm, less than 50 nm, less than 20 nm, less than 15 nm, less than 10 nm, or less than 5 nm.

As used herein, "applying" a composition or product refers to contacting it with a target or site such that the composition or product can have an effect on that target or site. The effect can be due to, for example, microbial growth and/or the action of a biosurfactant or other growth by-product. For example, the microbe-based compositions or products can be contacted with ore by pouring and/or spraying onto the ore.

As used herein, the terms "valuable minerals" and "valuable metals" refer to any mineral or metal that is extracted or mined from the earth, which has some economic value. The value of the mineral and/or metal is typically measured by how abundant or rare it is, with rarer minerals and/or metals having a higher economic value per unit of weight over those that are more abundant.

"Precious" or "rare" metals refer to naturally occurring metallic chemical elements having the highest economic value per unit of weight due to their extreme rarity. Precious metals include rhodium, platinum, gold, palladium, indium, iridium osmium, rhenium, ruthenium and silver. (Biltmore Loan and Jewelry 2016).

As used herein, "ore" refers to a naturally occurring solid material from which a valuable mineral and/or metal can be profitably extracted. Ores are often mined from ore deposits, which comprise ore minerals containing the valuable substance. "Gangue" minerals are minerals that occur in the deposit but do not contain the valuable substance. Examples of ore deposits include hydrothermal deposits, magmatic deposits, laterite deposits, volcanogenic deposits, metamorphically reworked deposits, carbonatite-alkaline igneous related deposits, placer ore deposits, residual ore deposits, sedimentary deposits, sedimentary hydrothermal deposits and astrobleme-related deposits. Ores, as defined herein, however, can also include ore concentrates or tailings, coal or coal waste products, or even other sources of metal or valuable minerals, including but not limited to, jewelry, electronic scraps, batteries and other scrap materials.

The transitional term "comprising," which is synonymous with "including," or "containing," is inclusive or open-ended and does not exclude additional, unrecited elements or method steps. By contrast, the transitional phrase "consisting of' excludes any element, step, or ingredient not specified in the claim. The transitional phrase "consisting essentially of' limits the scope of a claim to the specified materials or steps "and those that do not materially affect the basic and novel characteristic(s)" of the claimed invention. Use of the term "comprising" contemplates other embodiments that "consist" or "consist essentially of' the recited component(s).

Unless specifically stated or obvious from context, as used herein, the term "or" is understood to be inclusive. Unless specifically stated or obvious from context, as used herein, the terms "a," "and" and "the" are understood to be singular or plural.

Unless specifically stated or obvious from context, as used herein, the term "about" is understood as within a range of normal tolerance in the art, for example, within 2 standard deviations of the mean. "About" can be understood as within 10%, 9%, 8%, 7%, 6%, 5%, 4%, 3%, 2%, 1%, 0.5%, 0.1%, 0.05%, or 0.01% of the stated value.

Ranges provided herein are understood to be shorthand for all of the values within the range. For example, a range of 1 to 20 is understood to include any number, combination of numbers, or sub-range from the group consisting 1, 2, 3, 4, 5, 6, 7, 8, 9, 10, 11, 12, 13, 14, 15, 16, 17, 18, 19, 20, as well as all intervening decimal values between the aforementioned integers such as, for example, 1.1, 1.2, 1.3, 1.4, 1.5, 1.6, 1.7, 1.8, and 1.9. With respect to sub-ranges, "nested sub-ranges" that extend from either end point of the range are specifically contemplated. For example, a nested sub-range of an exemplary range of 1 to 50 may comprise 1 to 10, 1 to 20, 1 to 30, and 1 to 40 in one direction, or 50 to 40, 50 to 30, 50 to 20, and 50 to 10 in the other direction.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

Other features and advantages of the invention will be apparent from the following description of the preferred embodiments thereof, and from the claims. All references cited herein are hereby incorporated by reference.

### Biological Leaching Reagent

In specific embodiments, the subject invention provides microbe-based compositions and methods for recovering valuable minerals and/or metals, such as, e.g., gold, copper, silver, lithium and cobalt, from ore and/or mine tailings. These compositions, and the methods of their use are safe, environmentally-friendly and cost-efficient.

In preferred embodiments, the microbe-based composition of the subject invention is a biological leaching reagent comprising one or more microorganisms and/or microbial growth by-products. In one embodiment, at least one of the microorganisms is a biosurfactant-producing yeast.

The microorganisms in the microbe-based product may be in an active or inactive form, in spore form, mycelial form, or any other form of microbial propagule. Typically, the microorganism is inactive at the time it is applied to a site.

In one embodiment, the microbial growth by-product is a biosurfactant, enzyme, protein, peptide, amino acid and/or solvent. In one embodiment, the microbial growth by-products are biosurfactants. Biosurfactants according to the subject invention include, for example, low-molecular-weight glycolipids, cellobiose lipids, lipopeptides, flavolipids, phospholipids, and high-molecular-weight polymers such as lipoproteins, lipopolysaccharide-protein complexes, and/or polysaccharide-protein-fatty acid complexes.

In some embodiments, the growth by-product is produced by the one or more microorganisms present in the composition. In some embodiments, the growth by-product is added to the composition, either in crude or purified form, in addition to any growth by-products that are produced by the microorganisms.

In certain specific embodiments, the biological leaching reagent comprises a yeast fermentation product comprising yeast cell biomass and growth by-products thereof in fermentation medium in which the yeast was produced. Preferably, the yeast is a biosurfactant-producing yeast. Even more preferably, the yeast is *Starmerella bombicola,* which is capable of producing glycolipid biosurfactants, e.g., sophorolipids (SLP), at high concentrations.

In some embodiments, the yeast fermentation product is obtained during production of biosurfactants. During submerged cultivation of a biosurfactant-producing microorganism, biosurfactants are excreted into the fermentation broth. The biological leaching reagent can comprise the entire broth containing microbes, biosurfactants and other growth by-products, such as, e.g., excreted metabolites and/or cell wall components.

Alternatively, the biosurfactants can be harvested from the broth for further processing and/or purification. In one embodiment, *S. bombicola* produces a layer of SLP sediment in the culture comprising about 10-15% SLP, or about 4-5 g/L. In a specific embodiment, cultivation of the yeast occurs at 25-28 °C for 1 to 10 days. Advantageously, once the SLP layer is harvested from the culture, about 1-4 g/L of SLP can still remain in the supernatant, as well as yeast cell biomass and other advantageous yeast growth by-products and cellular components. In certain embodiments, the biological leaching reagent of the subject invention comprises the supernatant.

In certain embodiments, use of yeast fermentation products in the biological leaching reagents can be superior to, for example, purified microbial metabolites alone, due to, for example, the advantageous properties of yeast cell and/or cell walls. These properties include, for example, high concentrations of mannoprotein and/or beta-glucan in and/or on the yeast cell wall. These compounds can serve as, for example, effective emulsifiers. Additionally, the yeast fermentation product can further comprise biosurfactants, other metabolites, and/or cellular or extracellular components that are present in the culture, such as, e.g., solvents, acids, vitamins, minerals, enzymes, proteins, peptides, amino acids and others (e.g., lactic acid, ethanol, etc.), in the culture.

In one embodiment, the biological leaching reagent can further comprise nutrient sources, including sources of nitrogen, nitrate, phosphorus, magnesium and/or carbon.

In one embodiment, the biological leaching reagent can be enhanced with additional components as are needed, depending upon, for example, the ore type, mineral type, volume of ore, and other factors. These enhancing components can include additional microbial cultures, such as yeast and/or bacterial cultures. The enhancing components can also include additional pure or crude form biosurfactants, acids, solvents, enzymes, proteins, peptides and/or amino acids.

In some embodiments, the additional microbial cultures comprise biosurfactant-producers, such as, for example, *Wickerhamomyces anomalus, Pseudozyma aphidis, Saccharomyces cerevisiae, Pichia guilliermondii, Bacillus subtilis, Bacillus amyloliquefaciens, Bacillus licheniformis, Pseudomonas aeruginosa, Streptococcus* spp., and many others as are listed herein.

In some embodiments, the additional microbial cultures comprise microorganisms capable of accumulating nanoparticles of minerals and/or metals such as, for example, copper, cobalt, lithium, gold and/or silver, by solubilizing the metal present in ore to a soluble ionic form and converting it into nanoparticles within their cells. For example, thermophilic and/or acidophilic bacteria such as *Cupriavidus metallidurans,* which can precipitate nanoparticles of gold, can be added to the biological leaching reagent as an enhancement.

In some embodiments, the enhancing components comprise additional biosurfactants. The biosurfactants can be added as part of a microbial culture, or as a crude or purified form after being extracted from a microbial culture.

Biosurfactants are a structurally diverse group of surface-active substances produced by microorganisms. Biosurfactants are biodegradable and can be efficiently produced, according to the subject invention, using selected organisms on renewable substrates. Most biosurfactant-producing organisms produce biosurfactants in response to the presence of a hydrocarbon source (e.g. oils, sugar, glycerol, etc.) in the growing media. Other media components such as concentration of iron can also affect biosurfactant production significantly.

Microbial biosurfactants are produced by a variety of microorganisms such as bacteria, fungi, and yeasts. Exemplary biosurfactant-producing microorganisms include *Starmerella* spp. (e.g., *S. bombicola*), *Pseudomonas* spp. (e.g., *P. aeruginosa, P. putida, P. florescens, P. fragi, P. syringae*); *Flavobacterium* spp.; *Bacillus* spp. (e.g., *B. subtilis, B. amyloliquefaciens, B. pumillus, B. cereus, B. licheniformis*); *Wickerhamomyces* spp. (e.g., *W. anomalus*), *Candida* spp. (e.g., C. *albicans, C. rugosa, C. tropicalis, C. lipolytica, C. torulopsis); Saccharomyces* (e.g., *S. cerevisiae*); *Pseudozyma* spp. (e.g., *P. aphidis*); Rhodococcus spp. (e.g., *R. erythropolis*); *Arthrobacter* spp.; *Campylobacter* spp.; *Cornybacterium* spp.; *Pichia* spp. (e.g., *P. guilliermondii, P. occidentalis*); as well as others.

Biosurfactants are amphiphiles. They consist of two parts: a polar (hydrophilic) moiety and non-polar (hydrophobic) group. Due to their amphiphilic structure, biosurfactants increase the surface area of hydrophobic water-insoluble substances and increase the water bioavailability of such substances. Biosurfactants accumulate at interfaces, thus reducing interfacial tension and leading to the formation of aggregated micellar structures in solution.

The hydrocarbon chain of a fatty acid acts as the common lipophilic moiety of a biosurfactant molecule, whereas the hydrophilic part is formed by ester or alcohol groups of neutral lipids, by the carboxylate group of fatty acids or amino acids (or peptides), organic acid in the case of flavolipids, or, in the case of glycolipids, by the carbohydrate.

In certain embodiments, the biosurfactants according to the subject invention can comprise glycolipids, cellobiose lipids, lipopeptides, flavolipids, phospholipids, and polymers such as lipoproteins, lipopolysaccharide-protein complexes, and/or polysaccharide-protein-fatty acid complexes.

In some embodiments, the biosurfactants are glycolipids such as, for example, rhamnolipids (RLP), sophorolipids (SLP), trehalose lipids or mannosylerythritol lipids (MEL). In some embodiments, the biosurfactants are lipopeptides, such as, e.g., surfactin, iturin, fengycin, viscosin and/or lichenysin. In some embodiments, the biosurfactants are polymeric biosurfactants, such as, for example, emulsan, lipomanan, alasan, and/or liposan.

In one embodiment, the biological leaching reagent comprises, and/or is enhanced by the addition of, more than one biosurfactant and/or biosurfactant derivative. The biosurfactants may be mixed at any ratio as long as the composition contains the biosurfactants at concentration of 0.01 to 90%, preferably 0.05 to 50%, and more preferably 0.1 to 20%. In another embodiment, purified biosurfactants may be in combination with an accepted carrier, in that biosurfactants may be presented at concentrations of 0.0001 to 50% (v/v), preferably, 0.005 to 20% (v/v), more preferably, 0.001 to 5% (v/v).

In an exemplary embodiment, the biosurfactant is SLP. The SLP may be in a purified form or in crude form. The SLP may be added at concentrations of 0.01 to 90%, preferably 0.05 to 50%, and more preferably 0.1 to 20 wt %.

The microbe-based composition can comprise the fermentation medium containing the microorganism and/or the microbial metabolites produced by the microorganism and/or any residual nutrients. The product may be, for example, at least, by weight, 1%, 5%, 10%, 25%, 50%, 75%, or 99% growth medium. The amount of biomass in the product, by weight, may be, for example, anywhere from 0% to 100% inclusive of all percentages therebetween. For example, the biomass content of the fermentation broth may be, for example from 5 g/l to 180 g/l or more. In one embodiment, the solids content of the broth is from 10 g/l to 150 g/l.

Further components can be added to the microbe-based composition, for example, buffering agents, carriers, other microbe-based compositions produced at the same or different facility, viscosity modifiers, preservatives, nutrients for microbe growth, tracking agents, biocide, other microbes, surfactants, emulsifying agents, lubricants, solubility controlling agents, pH adjusting agents, stabilizers and ultra-violet light resistant agents.

In one embodiment, other leaching reagents can be added to the composition, or used in combination with it, for enhanced extraction of valuable minerals and/or metals. For example, acids, solvents, enzymes, proteins, peptides, sulfates and/or amino acids, produced by microbes or elsewhere, can be used, as well as other known leaching products. Preferably, any additional reagents are considered non-toxic and environmentally-friendly.

### Growth of Microbes

The subject invention provides methods for cultivation of microorganisms and production of microbial metabolites and/or other by-products of microbial growth. In one embodiment, the subject invention provides materials and methods for the production of biomass (e.g., viable cellular material), extracellular metabolites (e.g., small molecules and excreted proteins), residual nutrients and/or intracellular components (e.g., enzymes and other proteins).

The growth vessel used for growing microorganisms can be any fermenter or cultivation reactor for industrial use. In one embodiment, the vessel may have functional controls/sensors or may be connected to functional controls/sensors to measure important factors in the cultivation process, such as pH, oxygen, pressure, temperature, agitator shaft power, humidity, viscosity and/or microbial density and/or metabolite concentration.

In a further embodiment, the vessel may also be able to monitor the growth of microorganisms inside the vessel (e.g., measurement of cell number and growth phases). Alternatively, a daily sample may be taken from the vessel and subjected to enumeration by techniques known in the art, such as dilution plating technique. Dilution plating is a simple technique used to estimate the number of microbes in a sample. The technique can also provide an index by which different environments or treatments can be compared.

In one embodiment, the method includes supplementing the cultivation with a nitrogen source. The nitrogen source can be, for example, potassium nitrate, ammonium nitrate ammonium sulfate, ammonium phosphate, ammonia, urea, and/or ammonium chloride. These nitrogen sources may be used independently or in a combination of two or more.

The method can provide oxygenation to the growing culture. One embodiment utilizes slow motion of air to remove low-oxygen containing air and introduce oxygenated air. In the case of submerged fermentation, the oxygenated air may be ambient air supplemented daily through mechanisms including impellers for mechanical agitation of the liquid, and air spargers for supplying bubbles of gas to the liquid for dissolution of oxygen into the liquid.

The method can further comprise supplementing the cultivation with a carbon source. The carbon source is typically a carbohydrate, such as glucose, sucrose, lactose, fructose, trehalose, mannose, mannitol, and/or maltose; organic acids such as acetic acid, fumaric acid, citric acid, propionic acid, malic acid, malonic acid, and/or pyruvic acid; alcohols such as ethanol, isopropyl, propanol, butanol, pentanol, hexanol, isobutanol, and/or glycerol; fats and oils such as soybean oil, rice bran oil, canola oil, olive oil, corn oil, sesame oil, and/or linseed oil; etc. These carbon sources may be used independently or in a combination of two or more.

In one embodiment, the method comprises use of two carbon sources, one of which is a saturated oil selected from canola, vegetable, corn, coconut, olive, or any other oil suitable for use in, for example, cooking. In a specific embodiment, the saturated oil is 15% canola oil or discarded oil that has been used for cooking.

In one embodiment, the microorganisms can be grown on a solid or semi-solid substrate, such as, for example, corn, wheat, soybean, chickpeas, beans, oatmeal, pasta, rice, and/or flours or meals of any of these or other similar substances.

In one embodiment, growth factors and trace nutrients for microorganisms are included in the medium. This is particularly preferred when growing microbes that are incapable of producing all of the vitamins they require. Inorganic nutrients, including trace elements such as iron, zinc, copper, manganese, molybdenum and/or cobalt may also be included in the medium. Furthermore, sources of vitamins, essential amino acids, and microelements can be included, for example, in the form of flours or meals, such as corn flour, or in the form of extracts, such as yeast extract, potato extract, beef extract, soybean extract, banana peel extract, and the like, or in purified forms. Amino acids such as, for example, those useful for biosynthesis of proteins, can also be included.

In one embodiment, inorganic salts may also be included. Usable inorganic salts can be potassium dihydrogen phosphate, dipotassium hydrogen phosphate, disodium hydrogen phosphate, magnesium sulfate, magnesium chloride, iron sulfate, iron chloride, manganese sulfate, manganese chloride, zinc sulfate, lead chloride, copper sulfate, calcium chloride, calcium carbonate, sodium chloride and/or sodium carbonate. These inorganic salts may be used independently or in a combination of two or more.

In some embodiments, the method for cultivation may further comprise adding additional acids and/or antimicrobials in the liquid medium before and/or during the cultivation process. Antimicrobial agents or antibiotics are used for protecting the culture against contamination. Additionally, antifoaming agents may also be added to prevent the formation and/or accumulation of foam when gas is produced during cultivation.

The pH of the mixture should be suitable for the microorganism of interest. Buffers, and pH regulators, such as carbonates and phosphates, may be used to stabilize pH near a preferred value. When metal ions are present in high concentrations, use of a chelating agent in the liquid medium may be necessary.

The method and equipment for cultivation of microorganisms and production of the microbial by-products can be performed in a batch, quasi-continuous, or continuous processes.

In one embodiment, the method for cultivation of microorganisms is carried out at about 5° to about 100° C, preferably, 15 to 60° C, more preferably, 25 to 50° C. In a further embodiment, the cultivation may be carried out continuously at a constant temperature. In another embodiment, the cultivation may be subject to changing temperatures.

In one embodiment, the equipment used in the method and cultivation process is sterile. The cultivation equipment such as the reactor/vessel may be separated from, but connected to, a sterilizing unit, e.g., an autoclave. The cultivation equipment may also have a sterilizing unit that sterilizes in situ before starting the inoculation. Air can be sterilized by methods know in the art. For example, the ambient air can pass through at least one filter before being introduced into the vessel. In other embodiments, the medium may be pasteurized or, optionally, no heat at all added, where the use of low water activity and low pH may be exploited to control undesirable bacterial growth.

In one embodiment, the subject invention provides methods of producing a microbial metabolite by cultivating a microbe strain of the subject invention under conditions appropriate for growth and production of the metabolite; and, optionally, purifying the metabolite. In a specific embodiment, the metabolite is a biosurfactant. The metabolite may also be, for example, ethanol, lactic acid, beta-glucan, proteins, amino acids, peptides, metabolic intermediates, polyunsaturated fatty acids, and lipids. The metabolite content produced by the method can be, for example, at least 20%, 30%, 40%, 50%, 60%, 70 %, 80 %, or 90%.

The biomass content of the fermentation medium may be, for example from 5 g/l to 180 g/l or more. In one embodiment, the solids content of the medium is from 10 g/l to 150 g/l.

The microbial growth by-product produced by microorganisms of interest may be retained in the microorganisms or secreted into the growth medium. In another embodiment, the method for producing microbial growth by-product may further comprise steps of concentrating and purifying the microbial growth by-product of interest. In a further embodiment, the medium may contain compounds that stabilize the activity of microbial growth by-product.

In one embodiment, all of the microbial cultivation composition is removed upon the completion of the cultivation (e.g., upon, for example, achieving a desired cell density, or density of a specified metabolite). In this batch procedure, an entirely new batch is initiated upon harvesting of the first batch.

In another embodiment, only a portion of the fermentation product is removed at any one time. In this embodiment, biomass with viable cells remains in the vessel as an inoculant for a new cultivation batch. The composition that is removed can be a microbe-free medium or contain cells, spores, mycelia, conidia or other microbial propagules. In this manner, a quasi-continuous system is created.

Advantageously, the methods of cultivation do not require complicated equipment or high energy consumption. The microorganisms of interest can be cultivated at small or large scale on site and utilized, even being still-mixed with their media. Similarly, the microbial metabolites can also be produced at large quantities at the site of need.

### Microbial Strains

The microorganisms useful according to the subject invention can be, for example, bacteria, yeast and/or fungi. Preferably, the microorganisms are capable of producing, for example, biosurfactants, enzymes, proteins, peptides, amino acids and/or solvents.

These microorganisms may be natural, or genetically modified microorganisms. For example, the microorganisms may be transformed with specific genes to exhibit specific characteristics. The microorganisms may also be mutants of a desired strain. As used herein, "mutant" means a strain, genetic variant or subtype of a reference microorganism, wherein the mutant has one or more genetic variations (e.g., a point mutation, missense mutation, nonsense mutation, deletion, duplication, frameshift mutation or repeat expansion) as compared to the reference microorganism. Procedures for making mutants are well known in the microbiological art. For example, UV mutagenesis and nitrosoguanidine are used extensively toward this end.

In preferred embodiments, the microorganism is any yeast or fungus. Examples of yeast and fungus species suitable for use according to the current invention, include, but are not limited to, *Acaulospora, Aspergillus, Aureobasidium* (e.g., *A. pullulans*), *Blakeslea, Candida* (e.g., C. *albicans, C. apicola*), *Debaryomyces* (e.g., *D. hansenii*), *Entomophthora, Fusarium, Hanseniaspora* (e.g., *H. uvarum*), *Hansenula, Issatchenkia, Kluyveromyces, Mortierella, Mucor* (e.g., *M. piriformis*), *Penicillium, Phythium, Phycomyces, Pichia* (e.g., *P. anomala, P. guielliermondii, P. occidentalis, P. kudriavzevii*), *Pseudozyma* (e.g., *P. aphidis*), *Rhizopus, Saccharomyces* (*S. cerevisiae, S. boulardii sequela, S. torula*), *Starmerella* (e.g., *S. bombicola*), *Torulopsis, Thraustochytrium, Trichoderma* (e.g., *T. reesei, T. harzianum, T. virens*), *Ustilago* (e.g., *U. maydis*), *Wickerhamomyces* (e.g., *W. anomalus*), *Williopsis, Zygosaccharomyces* (e.g., Z. *bailii*).

In some embodiments, the microorganisms are bacteria, including Gram-positive and Gram-negative bacteria. Bacteria suitable for use according to the present invention include, for example, *Acinetobacter* (e.g., *A. calcoaceticus, A. venetianus*); *Agrobacterium* (e.g., *A. radiobacter*), *Azotobacter* (*A. vinelandii, A. chroococcum*), *Azospirillum* (e.g., *A. brasiliensis*), *Bacillus* (e.g., *B. amyloliquefaciens, B. finnus, B. laterosporus, B. licheniformis, B. megaterium, B. mucilaginosus, B. subtilis, B. coagulans* GBI-30 (BC30)), *Chlorobiaceae* spp., *Dyadobacter fermenters, Frankia* spp., *Frateuria* (e.g., *F. aurantia*), *Klebsiella* spp., *Microbacterium* (e.g., *M. laevaniformans*), *Pantoea* (e.g., *P. agglomerans*), *Pseudomonas* (e.g., *P. aeruginosa, P. chlororaphis, P. chlororaphis subsp. aureofaciens* (*Kluyver*), *P. putida*), *Rhizobium* spp., *Rhodospirillum* (e.g., *R. rubrum*), *Sphingomonas* (e.g., *S. paucimobilis*), and/or *Xanthomonas* spp.

In specific embodiments, the microorganism is the yeast *Starmerella bombicola,* which is an efficient producer of glycolipids, e.g., sophorolipids.

In some embodiments, the microorganism can be other biosurfactant- or other biochemical-producing microbes, such as, for example, *Wickerhamomyces anomalus, Pseudozyma aphidis, Saccharomyces cerevisiae, Pichia guilliermondii, Bacillus subtilis, Bacillus amyloliquefaciens, Bacillus licheniformis, Pseudomonas aeruginosa, Streptococcus* spp., and many others.

In some embodiments, the microorganism according to the subject invention is capable of solubilizing valuable minerals, such as, e.g., *Cupriavidus matallidurans,* which can accumulate nanoparticles of gold within its cells. Other examples of such microbes that are capable of reducing, oxidizing, and/or sequestering mineral components include, but are not limited to, *Bacillus* spp., *Sulfolobus* spp., *Thermoanaerobacter* spp., *Thiobacillus* spp., *Penicillium* spp., *Aspergillus* spp., *Sporosarcina* spp., *Pseudomonas* spp., *Pyrobaculum* spp., *Deinococcus geothermalis, Marinobacter pelagius,* and *Delftia acidovorans*

Other microbial strains can be used in accordance with the subject invention, including, for example, any other strains having high concentrations of mannoprotein and/or beta-glucan in their cell walls and/or that are capable of producing biosurfactants and other metabolites useful for sequestering, solubilizing and/or recovering minerals and metals from ore.

### Preparation of Microbe-based Products

The subject invention provides microbe-based products for use in recovering valuable minerals and/or metals from ore, as well as methods for removing impurities from ores. One microbe-based product of the subject invention is simply the fermentation medium containing the microorganism and/or the microbial metabolites produced by the microorganism and/or any residual nutrients. The product of fermentation may be used directly without extraction or purification. If desired, extraction and purification can be easily achieved using standard extraction and/or purification methods or techniques described in the literature.

The microbes and/or medium (e.g., broth or solid substrate) resulting from the microbial growth can be removed from the growth vessel and transferred for immediate use.

In one embodiment, the microbe-based product is simply the growth by-products of the microorganism collected from fermentation medium in crude form, comprising, for example, about 0.001% to 99% biosurfactant in liquid broth.

In one embodiment, the microbe-based product is a yeast fermentation product comprising a yeast strain and/or growth by-products thereof. The biological leaching reagent can comprise the entire fermentation broth containing microbes, biosurfactants and other growth by-products, such as, e.g., excreted metabolites and/or cell wall components.

In one embodiment, the yeast fermentation product can be obtained via submerged cultivation of a biosurfactant-producing yeast, e.g., *Starmerella bombicola.* This yeast is an effective producer of glycolipid biosurfactants, such as SLP. The fermentation broth after 5 days of cultivation at 25°C can contain the yeast cell suspension and, for example, 150 g/L or more of SLP.

Alternatively, the biosurfactants can be harvested from the broth for further processing and/or purification. In one embodiment, *S. bombicola* produces a layer of SLP sediment in the culture comprising about 10-15% SLP, or about 4-5 g/L. In a specific embodiment, cultivation of the yeast occurs at 25-28 °C for 1 to 10 days. Advantageously, once the SLP layer is harvested from the culture, about 1-4 g/L of SLP can still remain in the supernatant, as well as yeast cell biomass and other advantageous yeast growth by-products and cellular components.

The microorganisms in the microbe-based product may be in an active or inactive form. The microbe-based products may be used without further stabilization, preservation, and storage. Advantageously, direct usage of these microbe-based products preserves a high viability of the microorganisms (if live microbes are desired), reduces the possibility of contamination from foreign agents and undesirable microorganisms, and maintains the activity of the by-products of microbial growth.

In other embodiments, the composition (microbes, medium, growth by-products, or combinations thereof) can be placed in containers of appropriate size, taking into consideration, for example, the intended use, the contemplated method of application, the size of the fermentation tank, and any mode of transportation from microbe growth facility to the location of use. Thus, the containers into which the microbe-based composition is placed may be, for example, from 1 gallon to 1,000 gallons or more. In other embodiments the containers are 2 gallons, 5 gallons, 25 gallons, or larger.

Upon harvesting, for example, the yeast fermentation product, from the growth vessels, further components can be added as the harvested product is placed into containers and/or piped (or otherwise transported for use). The additives can be, for example, buffers, carriers, other microbe-based compositions produced at the same or different facility, viscosity modifiers, preservatives, nutrients for microbe growth, tracking agents, solvents, biocides, other microbes and other ingredients specific for an intended use.

Other suitable additives, which may be contained in the formulations according to the invention, include substances that are customarily used for such preparations. Examples of such additives include surfactants, emulsifying agents, lubricants, buffering agents, solubility controlling agents, pH adjusting agents, preservatives, stabilizers and ultra-violet light resistant agents.

In one embodiment, the product may further comprise buffering agents including organic and amino acids or their salts. Suitable buffers include citrate, gluconate, tartarate, malate, acetate, lactate, oxalate, aspartate, malonate, glucoheptonate, pyruvate, galactarate, glucarate, tartronate, glutamate, glycine, lysine, glutamine, methionine, cysteine, arginine and a mixture thereof. Phosphoric and phosphorous acids or their salts may also be used. Synthetic buffers are suitable to be used but it is preferable to use natural buffers such as organic and amino acids or their salts listed above.

In a further embodiment, pH adjusting agents include potassium hydroxide, ammonium hydroxide, potassium carbonate or bicarbonate, hydrochloric acid, nitric acid, sulfuric acid or a mixture.

In one embodiment, additional components such as an aqueous preparation of a salt as polyprotic acid such as sodium bicarbonate or carbonate, sodium sulfate, sodium phosphate, sodium biphosphate, can be included in the formulation.

Advantageously, in accordance with the subject invention, the microbe-based product may comprise broth in which the microbes were grown. The product may be, for example, at least, by weight, 1%, 5%, 10%, 25%, 50%, 75%, or 100% broth. The amount of biomass in the product, by weight, may be, for example, anywhere from 0% to 100% inclusive of all percentages therebetween.

Optionally, the product can be stored prior to use. The storage time is preferably short. Thus, the storage time may be less than 60 days, 45 days, 30 days, 20 days, 15 days, 10 days, 7 days, 5 days, 3 days, 2 days, 1 day, or 12 hours. In a preferred embodiment, if live cells are present in the product, the product is stored at a cool temperature such as, for example, less than 20° C, 15° C, 10° C, or 5° C. On the other hand, a biosurfactant composition can typically be stored at ambient temperatures.

### Methods of Extracting Valuable Minerals and/or Metals from Ore

The subject invention provides a method for extracting valuable minerals and/or metals from ore, wherein the method comprises applying a biological leaching reagent of the subject invention to the ore. The method can be used for bioleaching or for bio-stimulation of, for example, heap leaching and/or column leaching operations. In a preferred embodiment, the method is used for bioleaching of valuable minerals and/or metals from ore, for example, gold, silver, copper, cobalt, lithium, nickel and zinc.

In certain embodiments, the subject invention provides a method for extracting valuable minerals and/or metals from ore, wherein the method comprises obtaining ore from, e.g., an ore deposit, said ore comprising one or more valuable minerals and/or metals, in addition to gangue; applying a biological leaching reagent comprising one or more microorganisms and/or microbial growth by-products, to the ore; allowing the valuable minerals and/or metals to separate from the ore; and collecting the valuable minerals and/or metals.

In some embodiments, the ore particles are placed into a tank, column, vat or pool and the biological leaching reagent is applied to the ore particles by being poured into the tank, column, vat or pool.

The biological leaching reagent can enhance recovery of valuable minerals and/or metals from ore due to, for example, contact with microbial cells and/or their cell surface components, metal/mineral sequestration activity by microbial cells, and/or production of microbial metabolites such as, e.g., biosurfactants, solvents, enzymes, proteins, peptides and amino acids, that can help solubilize and/or disperse metal/mineral particles in liquid solution.

The microbes can be live (or viable), or inactive at the time of application. In certain embodiments, the microorganisms can grow *in situ* and produce active compounds (e.g., metabolites) onsite. Consequently, a high concentration of desirable metabolites (e.g., biosurfactants, solvents, enzymes, proteins, peptides and amino acids) and the microorganisms that produce them can be achieved easily and continuously at a treatment site (e.g., an ore mining site or a heap leaching pile).

The method can further comprise adding additional materials to enhance extraction of metals, for example, nutrients for microbial growth, additional microbial cultures, such as yeast and/or bacterial cultures and/or additional pure or crude form biosurfactants, acids, solvents, enzymes, proteins, peptides and/or amino acids.

In one embodiment, the ore has been previously mined from an ore deposit. In preferred embodiments, the mined ore is crushed, micronized, ground or pulverized into smaller ore particles prior to being contacted with the biological leaching reagent. Specifically, the ore can be crushed to a target maximum size of about 0.1 micron to about 1 inch in diameter, or about 0.5 micron to about 1 mm, or about 1 micron to about 100 microns. Methods and machinery for crushing ore are well known in the art.

In one embodiment, the ore is in the form of mine tailings, or the waste products left behind after a mineral has been separated from ore.

In a specific embodiment, the method comprises applying the biological leaching reagent in liquid form to the crushed ore particles, and mixing the particles and biological leaching reagent to form a liquid slurry.

The slurry can then be left for any amount of time sufficient to leach the valuable mineral and/or metal particles from the ore. The slurry can optionally be mixed and/or circulated continuously (e.g., mechanically or using aeration) throughout the leaching time period to ensure that maximum contact is made between the ore particles and the components of the biological leaching reagent, e.g., the yeast cell surfaces.

In one embodiment, the mineral particles are sequestered by the cells of the microorganism(s) of the biological leaching reagent. In one embodiment, the mineral particles separate from the ore and are dispersed and/or float in the liquid as solution. The liquid fraction of the slurry can be siphoned, drained, filtered, or otherwise removed. The mineral particles present in the liquid can be washed to remove residual microbial cell matter, collected, and dried, incinerated, and/or processed by any other means known in the metallurgical arts.

In some embodiments collecting or removing the separated particles is carried out using known methods, including, for example, gravity, froth flotation, electrostatic separation, magnetic separation, wet size screening, dry screening and cyclone classifying. It will be readily apparent to those skilled in the art that any other method for collecting the valuable minerals from the liquid slurry may be used.

With froth flotation, in particular, hydrophobicity differences between valuable metals/minerals and remaining ore components are increased through the use of biosurfactants and other metabolites produced by the microorganisms of the subject microbe-based compositions. The flotation process is used for the separation of a large range of sulfides, carbonates and oxides prior to further refinement.

In some embodiments, the subject methods comprise applying the biological leaching reagent in liquid form to a pile or column filled with crushed ore particles, and allowing the biological leaching reagent to percolate through the particles to a collection apparatus using gravity.

In some embodiments, the method can be used for bio-stimulation of heap leaching processes, wherein, after the ore has been mined and crushed or ground into small particles, the small particles are placed onto a heap leach pad to form a heap of ore particles; the biological leaching agent is poured and/or sprayed onto the heap; and the biological leaching reagent is allowed to percolate through the heap to the heap leach pad using gravity.

Examples of valuable metals and/or elements that can also be extracted using the methods of the subject invention, as well as valuable minerals that produce and/or comprise those metals and/or elements, include but are not limited to cobalt (e.g., erythrite, skytterudite, cobaltite, carrollite, linnaeite, and asbolite (asbolane)); copper (e.g., chalcopyrite, chalcocite, bornite, djurleite, malachite, azurite, chrysocolla, cuprite, tenorite, native copper and brochantite); gold (e.g., native gold, electrum, tellurides, calaverite, sylvanite and petzite); silver (e.g., sulfide argentite, sulfide acanthite, native silver, sulfosalts, pyrargyrite, proustite, cerargyrite, tetrahedrites); aluminum (e.g, bauxite, gibbsite, bohmeite, diaspore); antimony (e.g., stibnite); barium (e.g., barite, witherite); cesium (e.g., pollucite); chromium (e.g., chromite); iron (e.g., hematite, magnetite, pyrite, pyrrhotite, goethite, siderite); lead (e.g., galena, cerussite, anglesite); lithium (e.g., pegmatite, spodumene, lepidolite, petalite, amblygonite, lithium carbonate); magnesium (e.g., dolomite, magnesite, brucite, carnallite, olivine); manganese (e.g., hausmannite, pyrolusite, barunite, manganite, rhodochrosite); mercury (e.g., cinnabar); molybdenum (e.g., molybdenite); nickel (e.g., pentlandite, pyrrhotite, garnierite); phosphorus (e.g., hydroxylapatite, fluorapatite, chlorapatite); platinum group (platinum, osmium, rhodium, ruthenium, palladium) (e.g., native elements or alloys of platinum group members, sperrylite); potassium (e.g., sylvite, langbeinite); rare earth elements (cerium, dysprosium, erbium, europium, gadolinium, holmium, lanthanium, lutetium, neodymium, praseodymium, samarium, scandium, terbium, thulium, ytterbium, yttrium) (e.g., bastnasite, monazite, loparite); sodium (e.g., halite, soda ash); strontium (e.g., celestite, strontianite); sulfur (e.g., native sulfur, pyrite); tin (e.g., cassiterite); titanium (e.g., scheelite, huebnerite-ferberite); uranium (e.g., uraninite, pitchblende, coffinite, carnotite, autunite); vanadium; zinc (e.g., sphalerite, zinc sulfide, smithsonite, hemimorphite); and zirconium (e.g., zircon).

Additional elements and/or minerals, the extraction of which the subject invention is useful, include, e.g., arsenic, bertrandite, bismuthinite, borax, colemanite, kernite, ulexite, sphalerite, halite, gallium, germanium, hafnium, indium, iodine, columbite, tantalite-columbite, rubidium, quartz, diamonds, garnets (almandine, pyrope and andradite), corundum, barite, calcite, clays, feldspars (e.g., orthoclase, microcline, albite); gemstones (e.g., diamonds, rubies, sapphires, emeralds, aquamarine, kunzite); gypsum; perlite; sodium carbonate; zeolites; chabazite; clinoptilolite; mordenite; wollastonite; vermiculite; talc; pyrophyllite; graphite; kyanite; andalusite; muscovite; phlogopite; menatite; magnetite; dolomite; ilmenite; wolframite; beryllium; tellurium; bismuth; technetium; potash; rock salt; sodium chloride; sodium sulfate; nahcolite; niobium; tantalum and any combination of such substances or compounds containing such substances.

Advantageously, in certain embodiments, the methods take as little as a few hours, e.g., 3 to 12 hours, to one day to leach the minerals from the ore. The amount of time, however, depends upon, for example, how finely ground the ore particles are, the volume of ore particles being processed, and what types and/or combinations of microorganisms and other components are used in the biological leaching reagent.

Additionally, in one embodiment, the subject methods reduce the amount of refining and processing needed to recover pure or nearly pure metals from ore. For example, the subject invention can be used to separate the metals in a doré bar to reduce the amount of refining that is needed.

The method can be carried out at atmospheric pressure and lower temperatures than traditional metal smelting operations. Thus, the method does not require complicated equipment or high energy consumption, and cultivation of the biological leaching reagent used in the subject method can be performed on site, for example, at an ore mine or at a leaching site.

In one embodiment, the method can be used for removing a mineral or metal contaminant from water. Specifically, the method can be used to leach arsenic that has accumulated in water. For example, the method can comprise applying the biological leaching reagent to the contaminated water at a temperature greater than or equal to 40 °C. Advantageously, the arsenic accumulation in the water can be reduced by 50 to 90% using the subject method.

In one embodiment, the method can be used to absorb radioactive metals and to reduce the radioactivity thereof. For example, by applying the biological leaching reagent to pulverized radioactive ore, the method can be used for reducing the radioactivity of uranium, plutonium, radon, and other radioactive metals present in the ore.

The subject methods can be carried out at atmospheric pressure and lower temperatures than traditional metal smelting operations. Thus, the method does not require complicated equipment or high energy consumption, and cultivation of the biological leaching reagent used in the subject method can be performed on site, for example, at a mine or at a leaching site.

Advantageously, the present invention can be used without releasing large quantities of inorganic and toxic compounds into the environment. Additionally, the compositions and methods utilize components that are biodegradable and toxicologically safe, and can be used to reduce the amount of toxic waste produced during mining and leaching processes.

### Decadmiation Composition

In certain embodiments, the subject invention provides decadmiation compositions comprising components that are derived from microorganisms. In certain embodiments, the decadmiation composition comprises a microbial biosurfactant. In certain embodiments, the composition comprises a biosurfactant, an acid, and, optionally, a pH adjuster.

Use of the term "decadmiation" composition is not intended to limit the composition's usefulness to extracting or removing only cadmium from a substance. Rather, the subject invention further contemplates the ability of the composition to extract other metal impurities, such as, for example, lead, nickel, arsenic, copper, and vanadium.

In certain embodiments, the decadmiation composition comprises a microbe-based product comprising a biosurfactant utilized in crude form. The crude form can comprise, in addition to the biosurfactant, fermentation broth in which a biosurfactant-producing microorganism was cultivated, residual microbial cell matter or live or inactive microbial cells, residual nutrients, and/or other microbial growth by-products. The product may be, for example, at least, by weight, 1%, 5%, 10%, 25%, 50%, 75%, or 100% broth. The amount of biomass in the product, by weight, may be, for example, anywhere from 0% to 100% inclusive of all percentages therebetween.

In some embodiments, the biosurfactant is utilized after being extracted from a fermentation broth and, optionally, purified.

The biosurfactant according to the subject invention can be a glycolipid (e.g., sophorolipids, rhamnolipids, cellobiose lipids, mannosylerythritol lipids and trehalose lipids), lipopeptide (e.g., surfactin, iturin, fengycin, arthrofactin and lichenysin), flavolipid, phospholipid (e.g., cardiolipins), fatty acid ester compound, fatty acid ether compound, and/or high molecular weight polymers such as lipoproteins, lipopolysaccharide-protein complexes, and polysaccharide-protein-fatty acid complexes.

In certain specific embodiments, the biosurfactant is a sophorolipid (SLP), including linear SLP, lactonic SLP, acetylated SLP, de-acetylated SLP, salt-form SLP derivatives, esterified SLP derivatives, amino acid-SLP conjugates, and other SLP derivatives or isomers that exist in nature and/or are produced synthetically. In preferred embodiments, the SLP is a linear SLP or a derivatized linear SLP.

In some embodiments, the biosurfactant can be included in the composition at 0.01 to 99.9%, 0.1 to 90%, 0.5 to 80%, 0.75 to 70%, 1.0 to 50%, 1.5 to 25%, or 2.0 to 15% by weight, with respect to the total decadmiation composition.

In another embodiment, purified biosurfactants may be added in combination with an acceptable carrier, in that the biosurfactant may be presented at concentrations of 0.001 to 50% (v/v), preferably, 0.01 to 20% (v/v), more preferably, 0.02 to 5% (v/v).

In some embodiments, the biosurfactant can be included in the composition at, for example, 0.01 to 100,000 ppm, 0.05 to 10,000 ppm, 0.1 to 1,000 ppm, 0.5 to 750 ppm, 1.0 to 500 ppm, 2.0 to 250 ppm, or 3.0 to 100 ppm, with respect to the amount of phosphate ore being treated.

In certain embodiments, the acid of the decadmiation composition is an organic acid, such as, for example, acetic acid, citric acid, lactic acid, butyric acid, sorbic acid, benzoic acid, formic acid, fumaric acid, propionic acid, ascorbic acid, glyoxylic acid, malonic acid, pyruvic acid, oxalic acid, uric acid, malic acid, tartaric acid and/or analogs thereof. In certain embodiments, the acid is selected from inorganic acids such as, for example, sulfuric acid, nitric acid, phosphoric acid, hydrochloric acid, hydrofluoric acid, boric acid and analogs thereof. In preferred embodiments, the acid is citric acid.

In certain embodiments, the acid is characterized as a "weak" acid. Weak acids, as used herein, are acids that do not completely dissociate into ions in solution (i.e., less than 100% dissociation). Non-limiting examples of weak acids include citric acid, hydrosulfuric acid, hydrocyanic acid, nitrous acid, carbonic acid, sulfurous acid, phosphoric acid, hydrofluoric acid, benzoic acid, acetic acid, formic acid, and oxalic acid. Advantageously, in certain embodiments, the use of a weaker acid is effective for removal of impurities from an ore while retaining the integrity and value of the ore being treated.

In some embodiments, the acid can be included in the composition at 0.01 to 99.9%, 0.1 to 90%, 0.5 to 80%, 0.75 to 70%, 1.0 to 50%, 1.5 to 25%, or 2.0 to 15% by weight, with respect to the total decadmiation composition

In certain embodiments, the phosphate in the ore serves to buffer the acid and stabilize the formulation at pH 2 to 6, preferably about pH 4; however, optional additional pH adjusters can be included in the composition to adjust and/or stabilize the pH within the desired range.

In certain embodiments, the pH adjuster includes, but is not limited to, citrate, gluconate, tartarate, malate, acetate, lactate, oxalate, aspartate, malonate, glucoheptonate, pyruvate, galactarate, glucarate, tartronate, glutamate, glycine, pyridinium, lysine, glutamine, methionine, cysteine, arginine, sodium hydroxide, potassium hydroxide, ammonium hydroxide, ammonium chloride, formic acid, barium formate, potassium carbonate or bicarbonate, sodium phosphate, potassium phosphate, sodium chloride, Tris (trishydroxymethylaminomethane), sodium citrate, citric acid, sodium acetate, acetic acid, boric acid, borax, carbonic acid, sodium carbonate, hydrochloric acid, hydrofluoric acid, sodium fluoride, nitric acid, sulfuric acid, ammonia, and mixtures thereof.

In some embodiments, the pH adjuster can be included in the composition at 0.01 to 99.9%, 0.1 to 90%, 0.5 to 80%, 0.75 to 70%, 1.0 to 50%, 1.5 to 25%, or 2.0 to 15% by weight, with respect to the total decadmiation composition.

The decadmiation composition can further comprise other additives such as, for example, carriers, other microbe-based compositions, additional biosurfactants or chemical surfactants, enzymes, catalysts, solvents, salts, buffers, chelating agents, acids, emulsifying agents, lubricants, solubility controlling agents, preservatives, stabilizers, ultra-violet light resistant agents, viscosity modifiers, preservatives, tracking agents, biocides, and other microbes and other ingredients specific for an intended use.

### Methods of Extracting Impurities from Ore

In certain embodiments, the subject invention provides a method for extracting metals and other impurities from ore, including elements selected from, for example, Cd, U, Ca, V, Ti, Sn, Sr, Ag, Mn, Si, Al, Mg, Na, Fe, K, Zn, Cr, Cl, Co, Ni, Cu, As, Se, Br, Rb, Zr, Mo, In, Sn, Sb, I, Cs, Ba, La, Hf, W, Hg, Th and Sc. In certain specific embodiments, the impurity is cadmium, nickel, arsenic, iron, vanadium, mercury, copper, or lead. In certain preferred embodiments, the impurity is cadmium; however, in some embodiments, more than one impurity is present, wherein the total content of each of the more than one impurity is reduced according to the subject methods.

Though labeled as "impurities," in some embodiments, these compounds may simply be of lesser importance and/or value than the ore from which they are extracted rather than being simply a waste product. For example, cadmium is an impurity in phosphate ore, but can be useful once extracted for the production of batteries.

Additionally, use of the term "phosphate ore" is not intended to limit the method's usefulness to extracting or removing an impurity only from phosphate ore. Rather, the subject invention further contemplates the ability of the composition to extract impurities from other ores, such as ores described elsewhere in this Description, wherein the primary goal is to improve the value of the ore itself as opposed to obtaining a valuable mineral from an ore to produce leftover materials having little to no additional value. As one additional example, iron impurities can be found in silica sands used for glass manufacture.

In certain embodiments, the method comprises (i) obtaining the ore, said ore comprising a desirable component and an impurity; (ii) contacting a decadmiation composition according to the subject invention with the ore for a period of time to yield a mixture comprising a treated ore and an impurity that has reacted with the decadmiation composition; and (iii) separating the reacted impurity and decadmiation composition from the mixture to obtain a reduced-impurity material containing the desirable component. Step (iii) can be repeated as many times as necessary to achieve a desired reduction in impurity content. In a specific embodiment, the ore is phosphate ore, and the desirable component is phosphate or phosphorus.

In certain embodiments, the time period of step (ii) is from 10 minutes to 48 hours, about 30 minutes to 40 hours, or preferably about 12 hours to 24 hours. In certain embodiments, step (ii) comprises applying a liquid form decadmiation composition to the ore to produce a liquid mixture, and stirring or otherwise agitating the liquid mixture for the period of time.

The method can be carried out in a heap leach pad, a column, or any other laboratory or industrial sized reactor.

In some embodiments, when step (ii) is carried out in liquid, the reacted impurity and decadmiation composition of step (iii) is present in the liquid phase and the phosphate ore remains a solid that is decanted and filtered out of the liquid.

In some embodiments, step (iii) comprises applying a washing fluid comprising water and, optionally, an organic solvent to the mixture under agitation (e.g., shaking or stirring) for a period of time (e.g., 10 to 60 minutes). The washing fluid will comprise the reacted impurity and decadmiation composition and the phosphate ore remains a solid that is decanted and filtered out of the fluid. In some embodiments, the solvent is an alcohol, such as ethanol.

In certain embodiments, the method comprises (a) obtaining the ore, said ore comprising a desirable material and an impurity; (b) preparing a slurry of the ore in water and maintaining the slurry under agitation; (c) applying a decadmiation composition according to the subject invention to the slurry under agitation (e.g., shaking or stirring) for a period of time to yield a mixture comprising a treated ore and an impurity that has reacted with the decadmiation composition; (d) halting the agitation and allowing the mixture to stand for another period of time, thereby causing the formation of a precipitate comprising the treated ore and an aqueous layer comprising the reacted impurity and decadmiation composition; and (e) separating the precipitate from the layer of water to obtain a reduced-impurity material containing the desirable component. Step (e) can be repeated as many times as necessary to achieve a desired reduction in impurity content.

In a specific embodiment, the ore is phosphate ore and the desirable component is phosphate or phosphorus.

In some embodiments, step (e) comprises applying a washing fluid comprising water and, optionally, an organic solvent to the mixture under agitation (e.g., shaking or stirring) for a period of time (e.g., 10 to 60 minutes). The washing fluid will comprise the reacted impurity and decadmiation composition and the ore remains a solid that is decanted and filtered out of the fluid. In some embodiments, the solvent is an alcohol, such as ethanol.

The methods of the subject invention can be carried out at ambient temperature, and/or at a temperature of about 15 to 50° C., about 20 to 40° C., about 20 to 35° C., about 20 to 30° C., about 25° C., about 40 to 120° C., about 50 to 100° C., about 60 to 100° C., about 70 to 100° C., about 80 to 100° C., or about 100° C. In certain embodiments, a temperature higher than ambient temperature can be provided using a microwave, ultrasound, induction heating, plasma, electricity, or any combination thereof.

The methods of the subject invention can be carried out at ambient pressure, and/or at a pressure of about 50 bars, 75 bar, 100 bars, or greater than 100 bars.

In certain embodiments, the amount of the decadmiation composition applied is about 0.1 to 15%, about 0.1 to 10%, about 0.1 to 5%, about 0.1 to 3%, about 0.1%, or about 1 vol % based on an amount of the material containing the phosphate or other desirable component(s).

In certain embodiments, when a washing fluid is utilized, the amount of organic solvent in the washing fluid is preferably about 0.1 to 15%, about 0.5 to 10%, about 1 to 8%, about 1 to 5%, or about 1 vol %.

In certain embodiments the methods of the subject invention result in at least 25% reduction in impurities content, preferably at least 50% reduction after one treatment. In some embodiments, the ore can be treated multiple times to further reduce the impurities content.

In certain embodiments, the decadmiation composition according to the subject invention is effective due to amphiphiles-mediated penetration of the ore with an acid. In some embodiments, the sophorolipid or other biosurfactant serves as a vehicle for facilitating the transport of acid molecules into nanoscale pores within the ore. For example, in some embodiments, a sophorolipid will for a micelle containing the acid, wherein the micelle is less than 100 nm, less than 50 nm, less than 25 nm, less than 15 nm or less than 10 nm in size. The small size and amphiphilic properties of the micelle allow for enhanced penetration into the ore so that greater contact can be made with impurities therein.

In one embodiment, the method comprises crushing, grinding or pulverizing the ore into smaller particles, for example, less than 500 nm in size, prior to treating with the decadmiation composition.

In some embodiments, the ore is obtained from an ore deposit in a raw form. This raw form can comprise additional materials, or gangue. Thus, in certain embodiments, the method can further comprise, after obtaining the ore, e.g., phosphate ore, subjecting the ore to one or more beneficiation processes to liberate the ore from excess gangue material. The one or more beneficiation processes can include, for example, comminution, scrubbing, washing, screening, flotation, and/or hydrocycloning.

In some embodiments, the method comprises oxidizing the organic matter present in the phosphate ore using, for example, hydrogen peroxide, prior to contacting the phosphate ore with the decadmiation composition.

In some embodiments, the method comprises applying the decadmiation composition to wet process phosphoric acid produced after phosphate ore has undergone acidulation (reaction with, for example, sulfuric acid). In certain embodiments, the decadmiation composition can react with dissolved cadmium and other impurities present in the wet process phosphoric acid, causing the impurities to precipitate out of the liquid for collection and removal.

Advantageously, in certain embodiments, the decadmiation composition according to the subject invention has comparable effectiveness to traditional chemical extracting agents, but with reduced negative environmental impacts. Additionally, the methods of the subject invention do not require complicated equipment or high energy consumption, and production of the decadmiation composition can be performed on site, for example, at an ore mine or at a leaching site. Furthermore, the reduced-impurity phosphate materials produced according to the subject invention can be useful for producing more environmentally-friendly, reduced-toxicity fertilizer and animal feed supplements.

### Local Production of Microbe-Based Products

In certain embodiments of the subject invention, a microbe growth facility produces fresh, high-density microorganisms and/or microbial growth by-products of interest on a desired scale. The microbe growth facility may be located at or near the site of application. The facility produces high-density microbe-based compositions in batch, quasi-continuous, or continuous cultivation.

The microbe growth facilities of the subject invention can be located at the location where the microbe-based product will be used (e.g., a mine). For example, the microbe growth facility may be less than 300, 250, 200, 150, 100, 75, 50, 25, 15, 10, 5, 3, or 1 mile from the location of use.

Because the microbe-based product can be generated locally, without resort to the microorganism stabilization, preservation, storage and transportation processes of conventional microbial production, a much higher density of microorganisms can be generated, thereby requiring a smaller volume of the microbe-based product for use in the on-site application or which allows much higher density microbial applications where necessary to achieve the desired efficacy. This allows for a scaled-down bioreactor (e.g., smaller fermentation vessel, smaller supplies of starter material, nutrients and pH control agents), which makes the system efficient and can eliminate the need to stabilize cells or separate them from their culture medium. Local generation of the microbe-based product also facilitates the inclusion of the growth medium in the product. The medium can contain agents produced during the fermentation that are particularly well-suited for local use.

Locally-produced high density, robust cultures of microbes are more effective in the field than those that have remained in the supply chain for some time. The microbe-based products of the subject invention are particularly advantageous compared to traditional products wherein cells have been separated from metabolites and nutrients present in the fermentation growth media. Reduced transportation times allow for the production and delivery of fresh batches of microbes and/or their metabolites at the time and volume as required by local demand.

The microbe growth facilities of the subject invention produce fresh, microbe-based compositions, comprising the microbes themselves, microbial metabolites, and/or other components of the medium in which the microbes are grown. If desired, the compositions can have a high density of vegetative cells or propagules, or a mixture of vegetative cells and propagules.

In one embodiment, the microbe growth facility is located on, or near, a site where the microbe-based products will be used (e.g., a mine), for example, within 300 miles, 200 miles, or even within 100 miles. Advantageously, this allows for the compositions to be tailored for use at a specified location. The formula and potency of microbe-based compositions can be customized for specific local conditions at the time of application, such as, for example, which ore type is being treated; what type of mineral is being extracted; and what mode and/or rate of application is being utilized.

Advantageously, distributed microbe growth facilities provide a solution to the current problem of relying on far-flung industrial-sized producers whose product quality suffers due to upstream processing delays, supply chain bottlenecks, improper storage, and other contingencies that inhibit the timely delivery and application of, for example, a viable, high cell-count product and the associated medium and metabolites in which the cells are originally grown.

Furthermore, by producing a composition locally, the formulation and potency can be adjusted in real time to a specific location and the conditions present at the time of application. This provides advantages over compositions that are pre-made in a central location and have, for example, set ratios and formulations that may not be optimal for a given location.

The microbe growth facilities provide manufacturing versatility by their ability to tailor the microbe-based products to improve synergies with destination geographies. Advantageously, in preferred embodiments, the systems of the subject invention harness the power of naturally-occurring local microorganisms and their metabolic by-products to improve leaching processes.

The cultivation time for the individual vessels may be, for example, from 1 to 7 days or longer. The cultivation product can be harvested in any of a number of different ways.

Local production and delivery within, for example, 24 hours of fermentation results in pure, high cell density compositions and substantially lower shipping costs. Given the prospects for rapid advancement in the development of more effective and powerful microbial inoculants, consumers will benefit greatly from this ability to rapidly deliver microbe-based products.

### EXAMPLES

A greater understanding of the present invention and of its many advantages may be had from the following examples, given by way of illustration. The following examples are illustrative of some of the methods, applications, embodiments and variants of the present invention. They are not to be considered as limiting the invention. Numerous changes and modifications can be made with respect to the invention.

### EXAMPLE 1 - FERMENTATION OF STARMERELLA BOMBICOLA FOR BIOSURFACTANT PRODUCTION IN A 2000L GALLON REACTOR

A large-scale, fully enclosed reactor is used. The reactor has a working volume of 1500L when growing *S. bombicola* for SLP production.

In some embodiments, the nutrients for SLP production are glucose, urea, yeast extract, canola oil, magnesium sulfate, and potassium phosphate.

The reactor is inoculated with 10 liters of liquid culture grown separately in inoculum reactors. The duration of the cultivation cycle for SLP production is up to 120 hours, at 25° C and pH 3.5, with sampling performed once a day.

The final concentration of SLP is 70 gallons, with SLP concentration of 300-400 g/L. The entire broth can be harvested, containing SLP and yeast cells, and used directly.

Alternatively, the SLP can be extracted from the final product and used with or without purification and/or concentration. The remaining supernatant with cell biomass can also be used, comprising 1-4 g/L of residual SLP.

### EXAMPLE 2-PRODUCTION OF LIPOPEPTIDES BY BACILLUS SPP.

Fermentation of *Bacillus* bacteria can be performed in a nutrient medium containing (g/L), for example:

| | |
|---|---|
| Glucose | 18 |
| Powder molasses | 2 |
| Sucrose | 1 |
| KH₂PO₄ | 0.5 |
| Na₂HPO₄·7H₂O | 2.1 |
| KCl | 0.1 |
| MgSO₄ | 0.5 |
| CaCl₂ | 0.05 |
| Urea | 2.5 |
| NH₄Cl | 1.24 |
| Yeast extract | 2 |
| Corn peptone | 0.5 |
| TekNova trace element (mL) | 1 |
| pH 6.8 | |

Temperature of cultivation is about 40 °C, pH stabilization is from 6.8-7.0, and DO stabilization is at 30% (concentration of oxygen in the air is taken as 100%). Duration of cultivation is 24-32 hours. The final concentration of bacterial culture is no less than 1×10⁹ CFU/ml. The concentration of lipopeptides is 5-10 g/L.

### REFERENCES

Blog, Gold/Silver/Rare Coins. *Precious Metals in Order of Value.* Biltmore Loan and Jewelry; [updated 21 Feb., 2016; accessed 20 Nov. 2018]. https://www.biltmoreloanandjewelry.com/blog/precious-metals-in-order-of-value/. (Biltmore Loan and Jewelry 2016).

## Claims

1. A decadmiation composition comprising a biosurfactant, an acid and, optionally, a pH adjuster, wherein the acid is selected from acetic acid, citric acid, lactic acid, butyric acid, sorbic acid, benzoic acid, formic acid, fumaric acid, propionic acid, ascorbic acid, glyoxylic acid, malonic acid, pyruvic acid, oxalic acid, uric acid, malic acid, tartaric acid, sulfuric acid, nitric acid, phosphoric acid, hydrochloric acid, boric acid and analogs thereof

2. The decadmiation composition of claim 1, wherein the biosurfactant is a glycolipid, lipopeptide, or phospholipid.

3. The decadmiation composition of claim 2, wherein the biosurfactant is a sophorolipid.

4. The decadmiation composition of claim 3, wherein the sophorolipid is a linear sophorolipid or a derivative of a linear sophorolipid.

5. The decadmiation composition of claim 1, wherein the pH adjuster is present in the composition at a concentration that stabilizes the pH of the composition between 2-5.

6. A method for extracting an impurity from an ore, the method comprising:
(i) obtaining the ore, said ore comprising a desirable component and an impurity;
(ii) contacting a composition comprising a sophorolipid biosurfactant, an acid, and, optionally, a pH adjuster, with the ore for a period of time to yield a mixture comprising a treated ore and an impurity that has reacted with the composition; and
(iii) separating the reacted impurity and composition from the mixture to obtain a reduced-impurity material containing the desirable component.

7. The method of claim 6, wherein the ore is phosphate ore, and wherein the desirable component is phosphate or phosphorus.

8. The method of claim 6, wherein the acid is selected from acetic acid, citric acid, lactic acid, butyric acid, sorbic acid, benzoic acid, formic acid, fumaric acid, propionic acid, ascorbic acid, glyoxylic acid, malonic acid, pyruvic acid, oxalic acid, uric acid, malic acid, tartaric acid, sulfuric acid, nitric acid, phosphoric acid, hydrochloric acid, boric acid and analogs thereof.

9. The method of claim 6, wherein the pH adjuster is present in the composition at a concentration that stabilizes the pH of the composition between 2-5.

10. The method of claim 6, wherein the pH adjuster is sodium hydroxide or potassium hydroxide.

11. The method of claim 6, further comprising crushing, grinding or pulverizing the ore into particles less than 500 nm in size prior to step (ii).

12. The method of claim 6, wherein the composition is in liquid form, and wherein step (ii) comprises stirring or shaking the mixture for a time period of 10 minutes to 48 hours, and wherein the reacted impurity and composition of step (iii) is present in the liquid phase and the ore remains a solid that is decanted and filtered out of the liquid.

13. The method of claim 6, wherein step (iii) comprises applying a washing fluid comprising water and, optionally, an organic solvent to the mixture under agitation for a period of time or about 10 to 60 minutes, wherein the washing fluid will comprise the reacted impurity and composition and the ore remains a solid that is decanted and filtered out of the fluid.

14. The method of claim 13, wherein the solvent is an alcohol.

15. The method of claim 6, wherein the impurity is selected from cadmium, nickel, arsenic, iron, vanadium, mercury, copper, and lead.

16. The method of claim 6, wherein the impurity is cadmium.

17. A method for extracting an impurity from phosphate ore, the method comprising:
(a) obtaining the phosphate ore, said ore comprising phosphate and an impurity;
(b) preparing a slurry of the phosphate ore in water and maintaining the slurry under agitation;
(c) applying a composition comprising a sophorolipid biosurfactant, an acid, and, optionally, a pH adjuster, to the slurry under agitation for a period of time to yield a mixture comprising a treated phosphate ore and an impurity that has reacted with the composition;
(d) halting the agitation and allowing the mixture to stand for another period of time, thereby causing the formation of a precipitate comprising the treated phosphate ore and an aqueous layer comprising the reacted impurity and composition; and
(e) separating the precipitate from the layer of water to obtain a reduced-impurity phosphate-containing material.

18. The method of claim 17, wherein the acid is selected from acetic acid, citric acid, lactic acid, butyric acid, sorbic acid, benzoic acid, formic acid, fumaric acid, propionic acid, ascorbic acid, glyoxylic acid, malonic acid, pyruvic acid, oxalic acid, uric acid, malic acid, tartaric acid, sulfuric acid, nitric acid, phosphoric acid, hydrochloric acid, boric acid and analogs thereof.

19. The method of claim 17, wherein the pH adjuster is present in the composition at a concentration that stabilizes the pH of the composition between 2-5.

20. The method of claim 17, wherein the pH adjuster is sodium hydroxide or potassium hydroxide.

21. The method of claim 17, further comprising crushing, grinding or pulverizing the phosphate ore into particles less than 500 nm in size prior to step (b).

22. The method of claim 17, wherein step (e) comprises applying a washing fluid comprising water and, optionally, an organic solvent to the mixture under agitation for a period of time or about 10 to 60 minutes, wherein the washing fluid will comprise the reacted impurity and composition and the phosphate ore remains a solid that is decanted and filtered out of the fluid.

23. The method of claim 22, wherein the solvent is an alcohol.

24. The method of claim 17, further comprising using the reduced-impurity phosphate-containing material to produce a fertilizer or animal feed supplement.

25. The method of claim 17, wherein the impurity is selected from cadmium, nickel, arsenic, iron, vanadium, mercury, copper, and lead.

26. The method of claim 17, wherein the impurity is cadmium.
